(19) Europäisches Patentamt
European Patent Office
Office européen des brevets

(11) EP 1 569 672 B1

(12) # FASCICULE DE BREVET EUROPEEN

(45) Date de publication et mention de la délivrance du brevet:
**19.08.2009 Bulletin 2009/34**

(21) Numéro de dépôt: **03815106.4**

(22) Date de dépôt: **10.12.2003**

(51) Int Cl.:
***A61K 36/23*** *(2006.01)*

(86) Numéro de dépôt international:
**PCT/FR2003/003651**

(87) Numéro de publication internationale:
**WO 2004/062678 (29.07.2004 Gazette 2004/31)**

(54) **UTILISATION D'UN EXTRAIT DE CENTELLA ASIATICA RICHE EN MADECASSOSIDE ET EN TERMINOLOSIDE**

VERWENDUNG EINES CENTELLA ASIATICA EXTRAKTES , DER REICH AN MADECASSOSIDE UND TERMINOLOSIDE IST

USE OF A CENTELLA ASIATICA EXTRACT RICH IN MADECASSOSIDE AND IN TERMINOLOSIDE

(84) Etats contractants désignés:
**AT BE BG CH CY CZ DE DK EE ES FI FR GB GR HU IE IT LI LU MC NL PT RO SE SI SK TR**

(30) Priorité: **10.12.2002 FR 0215613**

(43) Date de publication de la demande:
**07.09.2005 Bulletin 2005/36**

(73) Titulaire: **Bayer Consumer Care AG**
**4052 Basel (CH)**

(72) Inventeurs:
- **LOISEAU, Alain**
  **64410 Bouillon (FR)**
- **SENE, Gérard**
  **75013 Paris (FR)**
- **THERON, Eric**
  **64121 Montardon (FR)**

(74) Mandataire: **Warcoin, Jacques et al**
**Cabinet Régimbeau**
**20, rue de Chazelles**
**75847 Paris Cedex 17 (FR)**

(56) Documents cités:
**EP-A- 0 867 447** **WO-A-01/19365**

- **DATABASE BIOSIS [Online] BIOSCIENCES INFORMATION SERVICE, PHILADELPHIA, PA, US; 1992, COLLINS D J ET AL: "TRITERPENE ACIDS FROM SOME PAPUA NEW GUINEA TERMINALIA SPECIES" XP002251030 Database accession no. PREV199293116315 & PHYTOCHEMISTRY (OXFORD), vol. 31, no. 3, 1992, pages 881-884, ISSN: 0031-9422**
- **DATABASE WPI Section Ch, Week 199239 Derwent Publications Ltd., London, GB; Class B04, AN 1992-321310 XP002251031 & KR 9 102 518 B (DONG KUK PHARM CO) 23 avril 1991 (1991-04-23)**
- **SAHU ET AL: 'Spectroscopic determination of structures of triterenoid trisaccharides from Centella asiatica' PHYTOCHEMISTRY vol. 28, no. 10, 1989, pages 2852 - 2854**
- **BRINKHAUS B. ET AL: 'Chemical, pharmacological and clinical profile of the East Asian medical plant Centella asiatica' PHYTOMEDICINE vol. 7, no. 5, 01 Octobre 2000, GUSTAV FISCHER VERLAG, STUTTGART, pages 427 - 448, XP001099148**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne un procédé de préparation d'un extrait comprenant un mélange de madécassoside, de terminoloside et le cas échéant d'asiaticoside, et l'utilisation d'un extrait de Centella asiatica comprenant un mélange de madécassoside et de terminoloside pour la fabrication d'un médicament ayant une activité anti-inflammatoire. Centella asiatica, encore connue sous le nom de Violette marronne (Ile de la Réunion) de Gotu Kola ou d'Indian Pennywort (Inde) ou de Centella repanda (Amérique du Nord) et de Talapetraka (Madagascar), est une herbe polymorphe qui pousse à l'état sauvage dans des régions humides et ombragées à une altitude idéale de 600 mètres.

Centella asiatica appartient à la famille des Ombellifères (Apiacées), particulièrement à la sous famille des Hydrocotyles. Centella correspond au nom du genre de la plante alors que asiatica correspond à son espèce. Centella asiatica inclut trois variétés dénommées Typica, Abyssinica et Floridana. Centella asiatica est connue et utilisée par les médecines traditionnelles malgache, indienne, chinoise, amérindienne ou indonésienne depuis plus de 3000 ans. Elle présente des utilisations variées et diverses selon les pays. Elle est particulièrement intéressante pour ces propriétés cicatrisantes, sédatives, analgésiques, anti-dépressives, anti-virales et anti-microbiennes. Elle est généralement utilisée par voie topique ou par voie orale. Paradoxalement, l'apparition de Centella asiatica dans la médecine moderne occidentale fut tardive, puisqu'elle ne fit son entrée au Codex qu'en 1884, et que le premier extrait sec ne fut réalisé qu'en 1941.

Les actifs de Centella asiatica sont des triterpènes pentacycliques, qui se trouvent à l'état de génines, ce sont les acides asiatique (formule I) et madécassique (formule II), et d'hétérosides, ce sont l'asiaticoside (formule III) et le madécassoside (formule IV).

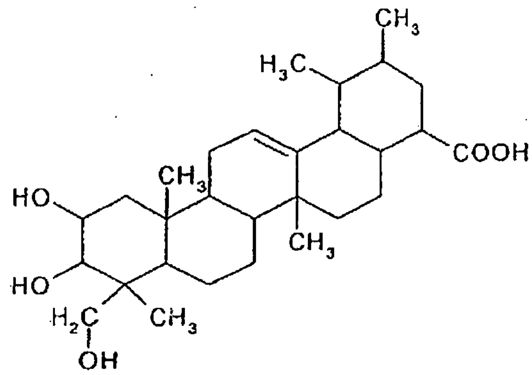

I

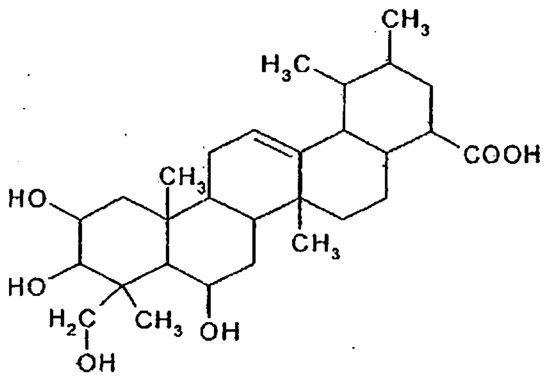

II

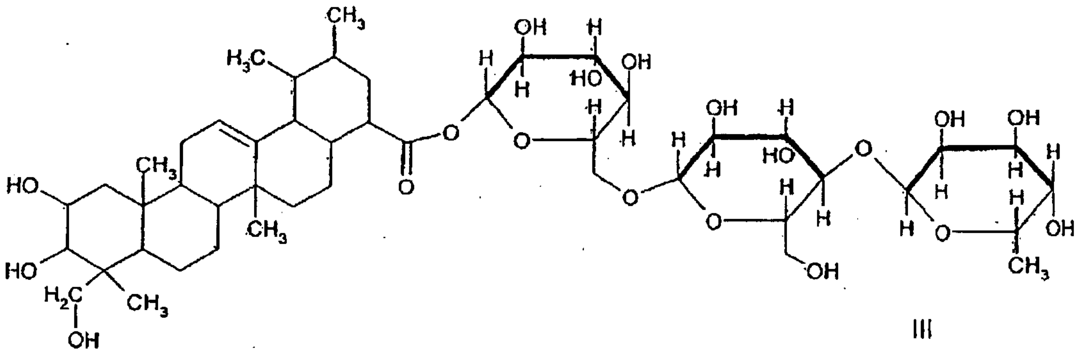

III

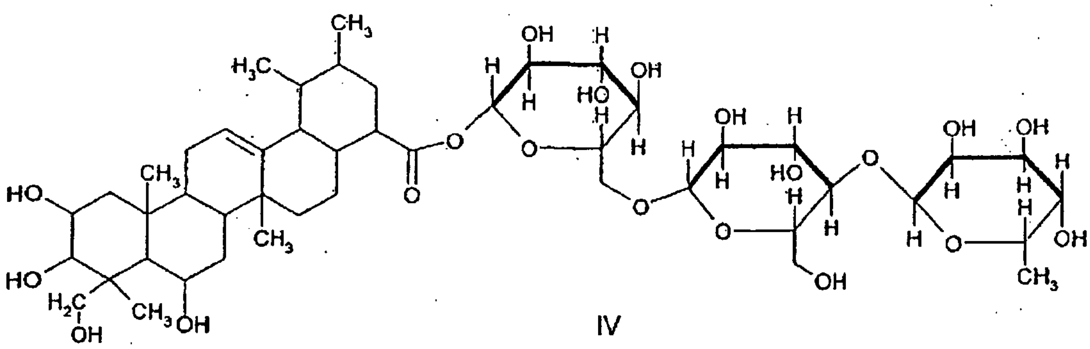

IV

Les molécules d'asiaticoside, de madécassoside, d'acide asiatique et d'acide madécassique participent aux défenses naturelles de la plante. Pour pouvoir exploiter industriellement les actifs de Centella asiatica de manière satisfaisante, il est donc indispensable de collecter la plante à l'état sauvage, un stress environnemental étant nécessaire pour que cette plante vivace présente une teneur en triterpènes importante.

Les hétérosides de Centella asiatica, madécassoside et asiaticoside, sont des complexes sucrés qui constituent les formes de réserve de l'acide madécassique et de l'acide asiatique de la plante, synthétisés essentiellement, en saison humide. L'agression de la plante par des bactéries, des levures ou des champignons active les hydrolases libératrices des génines. Les molécules triterpéniques sont particulièrement intéressantes du fait de leur action régulatrice et activatrice de la synthèse de collagènes. Les génines et hétérosides extraits de Centella asiatica favorisent notamment la synthèse des collagènes 1 et 3. Ces actifs sont utilisés dans le domaine pharmaceutique principalement pour faciliter la cicatrisation et dans le traitement de l'insuffisance veineuse. Ils sont utilisés dans le domaine cosmétique principalement en tant qu'agent anti-rides et anti-cellulite. Les actifs de Centella asiatica les plus couramment utilisées dans l'art antérieur sont les acides asiatique et madécassique, ainsi que l'asiaticoside. Le madécassoside étant très soluble dans l'eau, il est le plus souvent entraîné dans les eaux de lavage au cours des procédés d'extraction classiques des actifs liposolubles.

Les procédés de l'art antérieur permettent l'obtention d'un mélange d'asiaticoside et de madécassoside. Ce mélange comprend environ 25% en poids d'asiaticoside, 60% en poids de madécassoside et 15% en poids de produits secondaires, principalement constitués d'acides gras et d'osides, par rapport au poids total du mélange. D'autres procédés de l'art antérieur permettent aussi l'obtention de madécassoside pur à 81% en poids par rapport au poids total de l'extrait, ledit extrait comprenant en outre des isomères proches du madécassoside, des acides gras, principalement des acides linoléique, linolénique, palmitique ou oléique, et des sucres tels que des osides.

De manière surprenante, la demanderesse a mis au point un nouveau procédé d'extraction qui permet l'obtention d'un mélange de madécassoside, d'asiaticoside et d'une nouvelle molécule que l'on a appelé terminoloside, ledit mélange étant pur à plus de 75% en masse par rapport au poids total du mélange et l'obtention d'un mélange de madécassoside et de terminoloside obtenu pur à plus de 95% en masse par rapport au poids total du mélange. De la même manière, la demanderesse a découvert un extrait de Centella asiatica comprenant un mélange de madécassoside, de terminoloside et le cas échéant d'asiaticoside.

De manière surprenante, la demanderesse a également découvert que le mélange de madécassoside et de terminoloside extrait des parties aériennes de Centella asiatica pouvait être utilisé dans un médicament ayant une activité anti-inflammatoire.

En outre, le mélange de madécassoside, de terminoloside et d'asiaticoside extrait des parties aériennes de Centella asiatica peut être utilisé dans une composition cosmétique pour prévenir et retarder le vieillissement prématuré

de la peau.

Dans le cadre de la présente invention, on entendra par teminoloside la molécule chimique de nom général 2α, 3β, 6β, 23-tetrahydroxy oléa-12-ène 28-oate de 1[O-α-L-rhamnopyranosyl-(1-4)-O-β-glucopyranosol-(1-6)]-O-β-D-glucopyranose et de formule V suivante :

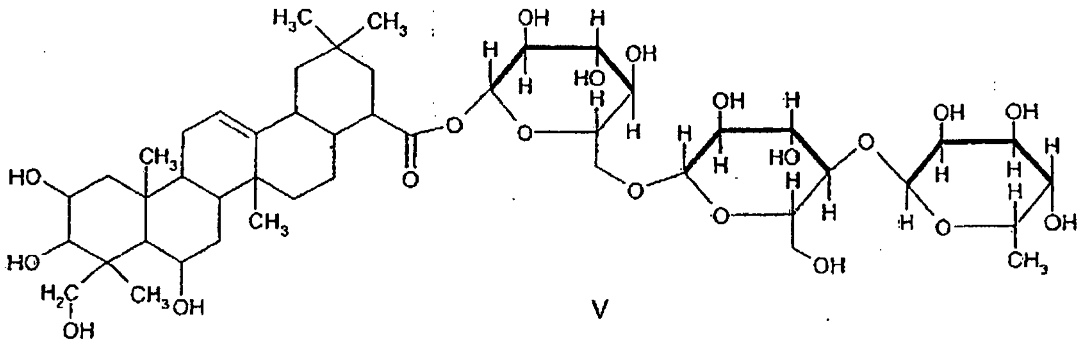

V

Cette molécule présente le même enchaînement sucré que le madécassoside, à savoir un enchaînement glucose-glucose-rhamnose. La structure du cycle terpénique du terminoloside correspond à celle du cycle terpénique de l'acide terminolique, de formule VI suivante :

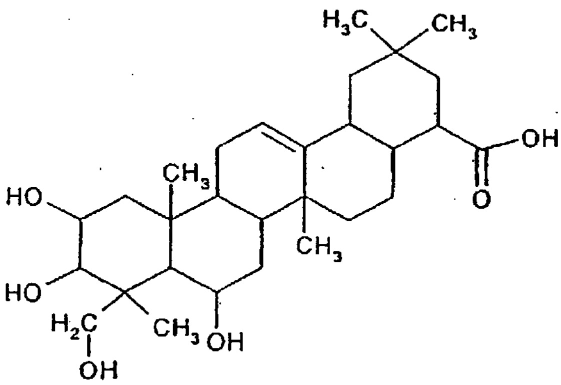

VI

Le terminoloside est ainsi un isomère de position du madécassoside.

Sahu et al. 1989, décrivent l'isolation de cette molécule dans un extrait de Centella asiatica.

On pourrait également supposer qu'il existe un isomère de l'asiaticoside non détecté. Ainsi, dans le cadre de la présente invention, on entend par le terme « asiaticoside » la molécule chimique de formule III éventuellement en mélange avec un de ses isomères oléaniques, s'il existe.

Dans le cadre de la présente invention, on désignera par l'expression « mélange binaire » un mélange comprenant principalement du madécassoside et du terminoloside, ce mélange ne comprenant quasiment pas d'asiaticoside et on désignera par l'expression « mélange ternaire » un mélange comprenant principalement du madécassoside, du terminoloside et de l'asiaticoside.

La présente invention a ainsi pour objet un procédé de préparation d'un extrait comprenant un mélange de madécassoside, de terminoloside et d'asiaticoside, caractérisé en ce qu'il comprend les étapes suivantes :

a) extraction des parties aériennes de Ccntella asiatica au moyen d'un solvant alcoolique ;
b) passage sur résine anionique de la solution alcoolique obtenue à l'étape a) ;
c) délipidation sélective par extraction liquide/liquide de l'éluat obtenu à l'étape b) ;
d) concentration de la phase hydro-alcoolique délipidée jusqu'en phase aqueuse avec filtrations successives ;
e) passage successif sur résine cationique puis sur résine anionique de la phase aqueuse obtenue à l'étape d) ;
f) stabilisation de la phase aqueuse obtenue à l'étape e) par addition d'alcool et obtention d'un mélange comprenant du madécassoside, du terminoloside et de l'asiaticoside.

La présente invention a pour objet un procédé de préparation d'un extrait comprenant un mélange de madécassoside et de Terminoloside, caractérisé en ce qu'il comprend les étapes suivantes :

a) extraction des parties aériennes de Centella asiatica au moyen d'un solvant alcoolique ;
b) passage sur résine anionique de la solution alcoolique obtenue à l'étape a) ;
c) délipidation sélective par extraction liquide/liquide de l'éluat obtenu à l'étape b) ;
d) concentration de la phase hydro-alcoolique délipidée jusqu'en phase aqueuse avec filtrations successives ;
e) passage successif sur résine cationique puis sur résine anionique de la phase aqueuse obtenue à l'étape d) ;
f) stabilisation de la phase aqueuse obtenue à l'étape e) par addition d'alcool ;
g) chromatographie sélective de la phase hydro-alcoolique pré-purifiée obtenue à l'étape f) ; et
h) récupération du mélange de madécassoside et de terminoloside dans sa forme finale.

L'extraction des principes actifs de la plante de Centella asiatica est réalisée avantageusement à partir des parties aériennes de la plante, dans le but principal de ne pas détériorer ses racines et ainsi de permettre le renouvellement naturel de cette plante vivace.

Dans une variante avantageuse du procédé selon l'invention, les parties aériennes sont mises à macérer dans un solvant alcoolique avant l'étape d'extraction. Les solvants alcooliques, qui peuvent être utilisés selon la présente invention, sont ceux traditionnellement usités par l'homme du métier, tel que l'éthanol et notamment l'éthanol à 70%. La solution alcoolique ainsi obtenue est ensuite purifiée au moyen d'un passage sur une résine anionique (étape b)). La résine anionique utilisée dans le procédé selon l'invention est avantageusement une résine anionique forte possédant des groupements fonctionnels du type ammonium quaternaire. Ce passage de la solution alcoolique sur une résine anionique permet le piégeage des substances anioniques secondaires, notamment des substances phénoliques.

Selon une variante avantageuse du procédé selon l'invention, la solution alcoolique obtenue suite à l'étape a) est clarifiée avant de passer sur la résine anionique. Cette étape avantageuse de clarification consiste à ajouter à ladite solution alcoolique une solution de base forte, telle qu'une solution d'hydroxyde de sodium, et du charbon activé. L'ajout de bases fortes permet la précipitation, et ainsi l'élimination par filtration, des métaux et des autres substances présentes susceptibles de réagir avec ces bases fortes. L'ajout de charbon activé permet en plus de décolorer, en la purifiant, la solution alcoolique, de fixer des acides gras ou des produits d'oxydation.

Suite à l'étape b) de passage sur résine, avantageusement précédée d'une étape de clarification, l'éluat obtenu est purifié de ces fractions grasses. Pour cela, il est soumis à une extraction liquide/liquide. Le solvant d'extraction utilisé est un solvant apolaire, avantageusement un alcane tel que l'heptane. Tous les procédés d'extraction liquide/liquide connus de l'homme du métier peuvent être mis en place dans le cadre du présent procédé selon l'invention. Notamment, ladite extraction peut être une centrifugation. Le madécassoside étant hydrosoluble, on récupère la phase hydro-alcoolique.

La phase hydro-alcoolique ainsi obtenue est ensuite concentrée jusqu'en phase aqueuse et filtrée successivement, avantageusement plusieurs fois de suite, pour permettre la précipitation des composés insolubles dans la phase aqueuse.

Suite à ces premières étapes du procédé selon l'invention, on obtient une phase aqueuse dans laquelle les deux hétérosides connus de Centella asiatica, à savoir l'asiaticoside et le madécassoside, ainsi que le terminoloside sont solubles. Cette phase aqueuse est ensuite purifiée par passage successif sur une résine cationique puis sur une résine anionique. Cette étape de passage successif sur résine cationique puis sur résine anionique est une étape essentielle du procédé selon l'invention. Avantageusement, la résine cationique utilisée est une résine cationique forte possédant des groupements fonctionnels du type sulfonates. De manière avantageuse, la résine anionique utilisée est une résine anionique forte possédant des groupements fonctionnels du type ammonium quaternaire. Dans le cadre du procédé selon l'invention, l'ordre des résines échangeuses d'ions est important afin d'améliorer la fixation de fractions acides résiduelles.

Selon une variante avantageuse de l'invention, suite aux passages successifs sur résine cationique puis anionique de l'étape e) et avant l'étape f), la phase aqueuse obtenue est aussi mis à passer au travers de charbon activé. Le charbon activé permet ainsi le captage des composés phénoliques secondaires responsables de la coloration de la solution.

Selon une autre variante avantageuse de l'invention, la phase aqueuse obtenue suite à l'étape e), avant l'étape f), et éventuellement suite à une étape de passage sur charbon activé, subit une ou plusieurs étape(s) de concentration.

La phase aqueuse obtenue suite à l'étape e), éventuellement après passage sur charbon activé et/ou décoloration, est ensuite stabilisée par ajout d'alcool (étape f)). On obtient alors une phase hydro-alcoolique pré-purifiée dans laquelle les deux hétérosides connus de Centella asiatica et le terminoloside sont solubles. Le mélange madécassoside, Terminoloside et asiaticoside est avantageusement obtenu avec une pureté supérieure à 75% en masse, par rapport au poids total dudit mélange.

La phase hydro-alcoolique pré-purifiée obtenue à l'étape f) comprenant le madécassoside, le terminoloside et l'asiaticoside peut ensuite être purifiée par chromatographie sélective. La technique de chromatographie sélective est bien connue de l'homme du métier. Elle permet de séparer les molécules selon leur affinité avec la phase stationnaire. Cette étape de chromatographie sélective permet de séparer l'asiaticoside du mélange constitué du madécassoside et

du terminoloside.

Dans le cadre de la présente invention, l'éluant utilisé lors de la chromatographie sélective est un solvant polaire. Avantageusement, le solvant est un mélange d'eau et d'éthanol dans des proportions en eau/éthanol variant de 50/50 à 90/10 et typiquement, 75/25 en volume /volume.

Dans le cadre de la présente invention, la phase stationnaire utilisée lors de la chromatographie sélective est une phase stationnaire apolaire. Avantageusement, la phase stationnaire est constituée de silices apolaires greffées. Les greffons apolaires ont avantageusement 2 à 18 atomes de carbone, et encore plus avantageusement 12 à 18 atomes de carbones.

Suite à ces étapes de procédé, c'est-à-dire à l'étape h), un mélange de madécassoside et de terminoloside est obtenu. Le mélange a avantageusement un rapport massique madécassoside :terminoloside compris entre 30 et 70%, plus avantageusement compris entre 40 et 60%. Ce mélange est avantageusement obtenu avec une pureté supérieure à 95% en masse par rapport au poids total dudit mélange. Ce mélange binaire ne comprend quasiment plus d'asiaticoside (seulement éventuellement en quantités de l'ordre des traces).

La présente invention a également pour objet l'utilisation d'un extrait de Centella asiatica susceptible d'être obtenu par le procédé tel que décrit précédemment et comprenant plus de 95% en masse d'un mélange de madécassoside et de terminoloside. Les impuretés présentes sont notamment des acides gras. Selon une variante avantageuse, le mélange de madécassoside et de terminoloside a un rapport massique en madécassoside par rapport au totum compris entre 30% et 70% en masse de madécassoside, avantageusement entre 40% et 60% en masse de madécassoside. Avantageusement, l'extrait est un agent anti-inflammatoire et immuno-modulateur.

Selon une autre variante avantageuse de l'invention, le procédé selon l'invention peut en outre comprendre un étape parallèle de standardisation du mélange de madécassoside, de terminoloside et d'asiaticoside (mélange ternaire), obtenu suite à l'étape f), par ajout d'une quantité appropriée d'un extrait très pur de Centella asiatica selon l'invention, c'est-à-dire d'un mélange de madécassoside et de terminoloside ayant une pureté supérieure à 95 % (mélange binaire), susceptible d'être obtenu par le procédé selon l'invention suite à l'étape h), de façon à ce que l'extrait final ainsi obtenu ait une pureté comprise entre 90 et 98% en poids, par rapport au poids total de l'extrait final. Le mélange ternaire est ainsi standardisé par addition du mélange binaire de façon à fixer l'extrait final ainsi obtenu dans une fourchette représentant une pureté située entre 90 et 98% en poids, par rapport au poids total de l'extrait final. Par exemple, pour obtenir un extrait final ayant une pureté de 90 % on mélangera 1 part du mélange binaire avec 1 part du mélange ternaire ; pour avoir une pureté finale de 98%, on mélangera 9 parts du mélange binaire avec 1 part du mélange ternaire.

La présente invention a également pour objet l'utilisation d'un extrait de Centella asiatica standardisé, susceptible d'être obtenu suite à l'étape de standardisation du procédé selon l'invention et comprenant au moins 75% en masse, avantageusement au moins 85% en masse, d'un mélange de madécassoside, de terminoloside et d'asiaticoside (mélange tertiaire standardisé). Les autres impuretés présentes sont notamment des acides gras. Dans cet extrait standardisé de Centella asiatica, le rapport massique asiaticoside :(madécassoside+terminoloside) est avantageusement compris entre 5 :95 et 25 :75. Si le pourcentage massique en asiaticoside est trop important, il y a un risque de séparation de phase. En effet l'asiaticoside est organosoluble alors que le madécassoside et le terminoloside sont hydrosolubles. Le mélange constitué de madécassoside et de terminoloside est tellement hydrosoluble qu'il peut solubiliser jusqu'à environ 25% en masse d'asiaticoside. Dans cet extrait standardisé de Centella asiatica, le rapport massique madécassoside: terminoloside est avantageusement compris entre 30:70 et 70:30, encore plus avantageusement entre 40:60 et 60:40.

La présente invention a aussi pour objet l'utilisation d'un médicament comprenant un extrait de Centella asiatica comprenant plus de 95% en masse d'un mélange de madécassoside et de terminoloside ou un extrait de Centella asiatica standardisé tels que décrits précédemment et un support pharmaceutiquement acceptable.

Le madécassoside et le terminoloside peuvent s'hydrolyser, notamment au contact de la flore cutanée, respectivement en acide madécassique et en acide terminolique. Cependant, ces acides respectifs ne montrent pas d'activité immuno-modulatrice.

Lorsqu'un corps étranger, tel qu'une bactérie ou un virus, pénètre dans notre organisme, ce dernier met en marche un système de défense, appelé système immunitaire, pour le combattre. Le système immunitaire doit éliminer le corps étranger sans pour autant détruire son propre organisme. Dans une maladie auto-immune, le système immunitaire ne reconnaît plus comme siennes certaines structures « de soi » de l'organisme, il se dérègle et devient capable d'agresser ces structures « de soi ».

Dans le cas particulier du psoriasis, les antigènes de l'organisme, circonvenus par la défense immunitaire, agressent la peau du sujet, ce qui entraîne une dérive inflammatoire. L'hyperréaction du système régulatoire entraîne une hyperprolifération des kératinocytes et donc la formation de plaques très épaisses et non homogènes de kératinocytes et de cornéocytes. Le médicament selon l'invention est destiné à inhiber la production pathologique des défenses immunitaires. Ledit médicament est avantageusement destiné à réguler les mécanismes inflammatoires. De manière encore plus avantageuse, ledit médicament est destiné au traitement des maladies auto-immunes, des maladies inflammatoires chroniques, des maladies inflammatoires atopiques ou des maladies intestinales. Avantageusement, ledit médicament est destiné au traitement du psoriasis, du vitiligo, du pityriasis, des sclérodermies, des dermatoses bulleuses,

de l'eczéma, de la dermatite atopique, de l'allergie ou de l'arthrite rhumatoïde.

Dans le domaine de la dermatologie, le médicament selon l'invention est aussi destiné à la prévention et au traitement des dérives inflammatoires chroniques liées au vieillissement et ses conséquences. Ledit médicament est avantageusement destiné à la prévention et au traitement des maladies choisies parmi les sensibilisations anaphylactiques, les anomalies pigmentaires de la peau, l'hypervascularisation dermique et les fissurations inflammatoires. Ledit médicament est aussi avantageusement destiné à réguler l'homéostasie tissulaire dermique, via une protection et une stimulation cellulaire ayant pour conséquence l'amélioration de la matrice extra-cellulaire contribuant au dialogue dermo-épidermique.

Le milieu extérieur agresse en permanence la peau, que ce soit via un rayonnement ultraviolet ou via le rayonnement émis par les lampes à décharge ou les divers antigènes atmosphériques naturels ou existants du fait de l'activité humaine, la pollution urbaine, etc., ce qui engage des processus biologiques d'accélération du vieillissement naturel. Le système anti-inflammatoire est ainsi en action de manière permanente, ce qui entraîne une accélération du renouvellement des kératinocytes de la peau, voir une hyperprolifération, aggravant l'entropie du tissu par une surexpression de protéines spécifiques et à terme une déperdition de fonctionnalité. La conséquence en est, un renouvellement épuisant des réserves naturelles de cellules de kératinocytes et l'entraînement d'un vieillissement prématuré de la peau.

Le médicament se présente avantageusement sous une forme solide, pâteuse ou liquide. Avantageusement selon la présente invention, le médicament est formulé pour être administré par voie topique, orale, sous-cutanée, injectable, rectale et génitale. De manière avantageuse selon la présente invention, lorsque le médicament est formulé pour être administré par voie topique, ledit médicament ou ladite composition se présente sous forme de solution aqueuse, de crème blanche ou colorée, de pommade, de lait, de lotion, de gel, d'onguent, de sérum, de pâte, de mousse, d'aérosol, de shampoing ou de stick. De manière avantageuse selon la présente invention, lorsque le médicament est formulé pour être administré par voie orale, ledit médicament peut se présenter sous la forme de solution aqueuse, émulsion, comprimés, gélules, capsules, poudres, granules, solutions ou encore de suspensions orales. De manière avantageuse selon la présente invention, lorsque le médicament est formulé pour être administré par voie sous-cutanée, ledit médicament ou ladite composition peut se présenter sous la forme d'ampoules injectables stériles. De manière avantageuse selon la présente invention, lorsque le médicament est formulé pour être administré par voie rectale, ledit médicament ou ladite composition peut se présenter sous la forme de suppositoires. De manière avantageuse selon la présente invention, lorsque le médicament est formulé pour être administré par voie génitale, ledit médicament peut se présenter sous la forme d'ovules.

La quantité du médicament selon l'invention à administrer dépend de la gravité et de l'ancienneté de l'affection traitée. Naturellement, le médecin adaptera aussi la posologie en fonction du malade. Le traitement de patients souffrant d'une maladie auto-immune, par l'extrait de Centella asiatica contenant plus de 95% en masse du mélange de madécassoside et de terminoloside par rapport au poids total du mélange, consiste notamment en l'administration topique dudit médicament à raison de 1 à 3% en poids, par rapport au volume de l'excipient, dudit extrait dans le véhicule transdermique, par jour en une ou plusieurs fois. D'une manière particulièrement avantageuse, l'administration dudit médicament à des doses telles que définies ci-dessus est répartie en 1 à 3 administrations quotidiennes.

Les exemples suivants illustrent l'invention sans toutefois en limiter la portée.

**Exemple 1: composition d'une crème selon l'invention**

Tableau 1

| Ingrédients : INCI | w/w % |
|---|---|
| Extrait madécassoside/terminoloside selon l'invention (pureté > 95%) | 1.0% |
| Beheneth - 10 | 1.5 % |
| Beheneth - 25 | 1.5% |
| Dycaprylyl Carbonate | 5.0% |
| Hexyl Laurate | 5.0% |
| Isohexadecane | 5.0% |
| Cetearyl Isononaoate | 5.0% |
| Dimethicone | 1.0% |
| Behenyl Alcohol | 2.0% |

(suite)

| Ingrédients : INCI | w/w % |
|---|---|
| Hydrogenated Vegetable Glycérides | 2.0% |
| Phenoxyethanol & parabens | 0.5% |
| Tocopheryl Acetate | 0.5% |
| Eau déminéralisée | qsp 100 |
| Glycerin | 3.0% |
| Butylen Glycol | 2.0% |
| Xanthan gum | 0.1% |
| Carbomer | 0.2% |

**Exemple 2: composition d'une crème selon l'invention**

Tableau 2

| Ingrédients : INCI | w/w % |
|---|---|
| Extrait madecassoside/terminoloside selon l'invention (pureté > 95%) | 1% |
| Cetearyl Glucoside & Cetearyl Alcohol | 5.0% |
| Caprylic/Capric Trygliceride | 5.0% |
| squalane | 5.0% |
| Cetearyl Isononanoate | 3.0% |
| Dimethicone crosspolymere | 2.0% |
| Stearyl Alcohol | 1.5% |
| Dycaprylyl Carbonate | 5.0% |
| Parabens | 0.1% |
| Eau déminéralisée | qsp 100 |
| Phenoxyethanol & Parabens | 0.5% |
| Glycerine | 2.0% |
| Carbomere | 0.3% |
| PEG 32 | 2.0% |
| Xanthan Gum | 0.2% |
| Aluminium starch octenylsuccinate | 2.0% |

**Exemple 3 : Procédé d'extraction d'un mélange de madécassoside et de terminoloside pur à plus de 95% en masse par rapport au poids total de l'extrait:**

**a) Défécation et purification sur colonne du jus alcoolique initial**

Les parties aériennes de Centella asiatica utilisées dans cet exemple comprennent les feuilles avec environ 2 à 3 cm de tige. 250 kg de parties aériennes de Centella asiatica sont séparés en trois lots de quantités égales. Les principes actifs de Centella asiatica sont extraits de ses parties aériennes selon le principe de l'extraction à contre-courant. Un lot de parties aériennes de Centella asiatica neuves, c'est-à-dire n'ayant encore subi aucune macération ni aucune filtration et donc très riche en principes actifs, est extrait une fois avec un jus de deuxième extraction, c'est-à-dire un solvant appauvri et presque saturé en principes actifs. Le jus ainsi obtenu, que l'on appelle jus final, est ensuite

directement envoyé dans une cuve de défécation. Le lot est ensuite soumis à une deuxième extraction avec un jus de première macération, c'est-à-dire un jus moins chargé que le jus utilisé précédemment et donc apte à solubiliser plus de principes actifs. C'est ce jus qui sert de jus de deuxième extraction. Le lot est enfin soumis à une troisième extraction avec un solvant neuf et ainsi apte à solubiliser les dernier pourcentages de principes actifs encore présents dans le lot. C'est ce jus qui sert de jus de première extraction.

Dans le cadre de cet exemple le solvant de macération et de filtration utilisé est de l'éthanol à 70%. Pour 250 kg de parties aériennes de Centella asiatica, on utilise 1600 L d'éthanol à 70%.

Suivant si l'étape précédente d'extraction est réalisée en cuve statique ou en cuve dynamique, le temps de macération des parties aériennes de Centella asiatica dans l'éthanol à 70%, plus ou moins saturé en principes actifs, varie de un jour à quelques heures. Dans le cas particulier de cuves dynamiques chauffées à une température de 60°C, le temps de macération est de deux heures. On ajoute ensuite au jus final 10 L d'une solution d'hydroxyde de sodium à 30 % puis 10 kg de charbon activé, qui permet par agitation de décolorer la solution. La solution est laissée sous agitation 40 minutes puis elle est filtrée. On passe le filtrat à travers une résine anionique forte, possédant des groupements fonctionnels du type ammonium quaternaire, à un débit de 300 L/h.

L'éluat hydro-alcoolique ainsi obtenu est ensuite neutralisé par l'hydroxyde de sodium à un pH apparent compris entre 5,3 et 6,9 puis soumis à une extraction liquide/liquide qui est avantageusement centrifuge, par de l'heptane.

La phase dégraissée ainsi obtenue est concentrée au moyen d'une évaporation continue sous pression réduite à une température ne dépassant pas 70°C. Cette concentration permet d'éliminer l'asiaticoside contenu dans la phase dégraissée. On concentre jusqu'à récupérer un volume d'éthanol correspondant à environ 90% en volume du volume initial de la phase dégraissée et jusqu'à la précipitation dans la phase dégraissée de l'asiaticoside. Cette phase est ensuite filtrée et on récupère les eaux mères. Le madécassoside est tellement hydrosoluble qu'il solubilise environ 20% en masse de l'asiaticoside ayant précipité lors de l'étape précédente de concentration.

**b) Etapes de préparation d'une phase hydro-alcoolique pré-purifiée**

La phase aqueuse concentrée récupérée est purifiée par passage sur résine cationique puis sur résine anionique. La résine cationique est une résine cationique forte possédant des groupements fonctionnels du type sulfonates. La résine anionique est une résine anionique forte possédant des groupements fonctionnels du type ammonium quaternaire. L'éluat ainsi obtenu est une phase hydroalcoolique qui est ensuite neutralisée par l'acide chlorhydrique à un pH compris entre 6,75 +/- 0,25 et décolorée par passage sur du charbon activé.

La solution ainsi obtenue est filtrée puis concentrée, en réacteur sous pression réduite, jusqu'à l'obtention d'une phase aqueuse selon le même principe que la concentration précédente. Elle est ensuite réajustée par ajout d'éthanol afin d'obtenir une solution hydro-alcoolique stabilisée ayant une teneur en matières sèches de 200 g +/- 20 par litre et un pourcentage d'alcool de 35% +/- 3,5 % volume / volume On obtient ainsi une phase pré-purifiée riche en madécassoside et en terminoloside et contenant encore de l'asiaticoside.

**c) Etape de purification**

La phase hydro-alcoolique pré-purifiée ainsi obtenue est ensuite sownise à une séparation par chromatographie sélective. Lors de cette chromatographie sélective, la phase stationnaire utilisée est une phase constituée de silices apolaires greffées, les greffons apolaires ayant 18 atomes de carbone et mesurant 10$\mu$m. Le solvant utilisé est un mélange d'eau à 65% en volume et d'éthanol à 35% en volume par rapport au volume total du mélange. L'éluat ainsi obtenu est ensuite concentré, séché, broyé. Ces différentes étapes permettent l'obtention d'un extrait sec de Centella asiatica.

L'extrait sec contient 99,8% en masse, par rapport au poids total de l'extrait, d'un mélange 51.49 en masse de madécassoside et de terminoloside (mélange binaire).

**<u>Exemple 4: Procédé d'extraction et de préparation d'un mélange standardisé de madécassoside, terminoloside et asiaticoside</u> :**

Le procédé d'extraction est identique à celui décrit dans l'exemple 3 jusqu'à l'étape d'obtention d'une phase pré-purifiée riche en madécassoside et en terminoloside et contenant encore de l'asiaticoside (avant l'étape de purification) : mélange ternaire.

Cette phase pré-purifiée a une pureté en madécassoside, terminoloside et asiaticoside de 80% en masse. Les impuretés présentes sont notamment des acides gras. Si on ramène les quantités de madécassoside, de terminoloside et d'asiaticoside à 100%, le mélange comprend environ 39% en poids de madécassoside, 39% en poids de terminoloside et 22% en poids d'asiaticoside.

Cet extrait est standardisé par ajout de 100% en masse du mélange binaire obtenu à l'exemple 3 (soit autant

de mélange ternaire que de mélange binaire).

On obtient alors un extrait standardisé de Centella asiatica présentant une pureté de 90% en masse. Cet extrait comprend, Si on ramène les quantités de madécassoside, de terminoloside et d'asiaticoside à 100%, 45,7% en poids de madécassoside, 44,5% en poids de terminoloside et 9,8% en poids d'asiaticoside.

**Exemple 5 : Isolement des deux isomères madécassoside et terminoloside:**

300 mg d'un mélange de madécassoside et terminoloside sont chromatographiés sur une colonne préparatrice (de marque Hypurity C18 (250 x 4,6)) ayant une phase mobile eau :acétonitrile 81 :19 v/v, à 30°C. La détection est effectuée à une longueur d'onde de 210 nm. On obtient ainsi 95 mg de terminoloside et 85 mg de madécassoside.

La structure chimique de madécassoside et de terminoloside est confirmée par analyse spectrométrique (RMN 1H, RMN 13C et spectrométrie de masse).

**Exemple 6: évaluation des effets modulateurs du mélange de madécassoside et de terminoloside vis-à-vis de la croissance cellulaire de kératinocytes humains hyperprolifératifs :**

Dans cet exemple ainsi que dans les exemples suivants, l'extrait contient 99.8 % du mélange de madécassoside et de terminoloside en poids selon l'invention par rapport au poids total de l'extrait ; ledit mélange contenant 51% en poids de madécassoside et 49% en poids de terminoloside, par rapport au poids total du mélange.

L'extrait selon l'invention contenant principalement le mélange de madécassoside et de terminoloside, ledit extrait sera indifféremment désigné, dans cet exemple ainsi que dans les exemples suivants, sous l'expression un mélange de madécassoside et de terminoloside ou sous l'expression un mélange selon l'invention.

Le sujet de cet exemple est d'évaluer les effets modulateurs du mélange de madécassoside et de terminoloside vis-à-vis de la croissance cellulaire des kératunocytes humains hyperprolifératifs. L'exemple a été réalisé sur des kératinocytes humains HaCaT stimulés par le Keratinocyte Growth Factor (ci après dénommé KGF) et/ou l'Elastase Humaine Leucocytaire (ci après dénommée EHL).

**a) Etude de la cytotoxicité du mélange de madécassoside et de terminoloside**

Cette étude de cytotoxicité consiste à déterminer la dose maximale de produit n'entraînant pas de toxicité cellulaire. La cytotoxicité a été étudiée sur des kératinocytes humains HaCaT ensemencés dans des plaques 96-puits à raison de $20\times10^3$, gamme large, et $10\times10^3$, gamme restreinte, cellules par puits. La viabilité cellulaire est évaluée par un test colorimétrique au Rouge Neutre. Le Rouge Neutre est un colorant faiblement cationique qui pénètre les membranes cellulaires par un phénomène de diffusion non ionique et qui se fixe au niveau intracellulaire sur les groupements phosphate et/ou carboxylique de la matrice lysosomiale.

Des modifications dans l'accumulation et la rétention du Rouge Neutre sont observées lorsque les membranes cellulaires sont endommagées. La densité optique de la solution obtenue après incubation des cellules en présence de Rouge Neutre est donc proportionnelle au nombre de cellules vivantes.

Une gamme large de 9 concentrations, de 0,001 à 1 mg/ml, a été testée dans un premier temps. Les différentes solutions-tests ont été préparées dans du milieu DMEM, Dubelco Modified Essential Meduim, additionné de 10% en volume de SVF, sérum de veau fatal. Après 24 heures de contact, les concentrations inférieures ou égales à 1 mg/ml n'induisent aucune modification significative de la réponse cellulaire vis-à-vis de Rouge Neutre. Une très faible diminution de la capture du Rouge Neutre, correspondant à 10% d'inhibition, est observée uniquement à la plus forte concentration testée de 1 mg/ml. A partir de ces résultats, une gamme restreinte, entre 0,05 et 5 mg/ml, a été établie afin de préciser la dose maximale non cytotoxique. La viabilité cellulaire est évaluée par un test colorimétrique au Rouge Neutre, après 72 heures d'incubation. Les résultats confirment la non toxicité des concentrations inférieures ou égales à 1mg/ml. A partir de 1 mg/ml, on observe un léger effet cytotoxique dose-dépendant. La plus forte concentration testée, 5 mg/ml, inhibe de 43% la réponse cellulaire vis-à-vis du Rouge Neutre.

Une seconde gamme restreinte comprise entre 0,075 et 5 mg/ml a été testée. L'essai a été réalisé sur des cellules HaCaT, après solubilisation du mélange de madécassoside et de terminoloside dans du diméthylsulfoxide, ci après dénommé DMSO, à une concentration de 1%. La viabilité cellulaire est évaluée par un test colorimétrique au Rouge Neutre, après 72 heures d'incubation. Les résultats obtenus sont similaires à ceux enregistrés en absence de DMSO.

Le tableau 3 ci-dessous regroupe l'ensemble des résultats, la viabilité cellulaire est exprimée en pourcentage de l'essai témoin.

Tableau 3

| Concentration (mg/ml) | 0,05 | 0,075 | 0,1 | 0,25 | 0,5 | 0,75 | 1 | 0,75 | 5 |
|---|---|---|---|---|---|---|---|---|---|
| Gamme large (24h) | 102% | n.t. | 105% | n.t. | 100% | n.t. | 90% | n.t. | n.t. |
| Gamme restreinte n°1 (72h) | 99% | 100% | 98% | 99% | 97% | 96% | 91% | 80% | 57% |
| Gamme restreinte n°2 (72h) | n.t. | 102% | 101% | 99% | 97% | 94% | 91% | 78% | 55% |
| L'abréviation n.t. signifie non testé. | | | | | | | | | |

Compte tenu de ces résultats, on a décidé de retenir la concentration de 300µg/ml, comme dose maximale, pour tester l'activité anti-prolifération du mélange de madécassoside et de terminoloside.

**b) Effet sur la prolifération cellulaire :**

Le principe du test repose sur l'évaluation de la croissance de kératinocytes humains hyperprolifératifs en absence et en présence du mélange de madécassoside et de terminoloside. La méthode est basée sur la mesure des densités cellulaires au niveau des cultures stimulées par le KGF ou par l'EHL. Les densités cellulaires sont appréciées par un test colorimétrique au Rouge Neutre. L'absorbance des solutions est lue à 540 nm à l'aide d'un lecteur de microplaque.

4 concentrations du mélange de madécassoside et de terminoloside ont été testées :

$C_1$=10 µg/ml $C_2$=30 µg/ml $C_3$=100 µg/ml $C_4$=300µg/ml

b.1) Stimulation par le KGF :

On prépare 6 lots tests : un lot contrôle (cellules non stimulées), un lot témoin KGF (cellules stimulées par KGF à une concentration de 100ng/ml) et 4 lots traités (cellules stimulées par KGF et traitées avec le mélange de madécassoside et de terminoloside). On mesure les densités otiques à t=0 jours, t=2 jours et t=5 jours. Les moyennes des densités optiques, D.O., sont converties en densités cellulaires, cellules/puits, à partir de la droite de régression :

$$D.O.=0{,}00355\times(10^3\ \text{cellules/puits})+0{,}0018$$

Le tableau 4 suivant regroupe les densités cellulaires enregistrés au bout de 2 et 5 jours après activation des cellules par le KGF.

Tableau 4

| | Traitement 2 jours | | Traitement 5 jours | |
|---|---|---|---|---|
| Stimulation KGF | Cellules/puits | Croissance cellulaire | Cellules/puits | Croissance cellulaire |
| Témoin sans KGF | 56465 +/- 2486 | | 115802 +/- 988 | |
| Témoin avec KGF | 85833 +/- 1742 | 100% | 157616 +/- 6359 | 100% |
| $C_1$ et KGF | 84388+/-2616 | 98% n.s. | 161354 +/- 5165 | 102% n.s. |
| $C_2$ et KGF | 85304 +/- 1312 | 99% n.s. | 161248 +/- 4232 | 102% n.s. |
| $C_3$ et KGF | 84317 +/- 1444 | 98% n.s. | 152081+/-2536 | 96% n.s. |
| $C_4$ et KGF | 83259 +/-1960 | 97% n.s. | 150071 +/- 7087 | 95% n.s. |
| L'abréviation n.s. signifie que le résultat n'est pas statistiquement significatif. | | | | |

Les résultats obtenus ne montrent aucune modification significative de la croissance des cellules HaCaT stimulées par le KGF, après 2 ou 5 jours de culture en présence du mélange selon l'invention.

b.2) Stimulation par l'EHL :

On prépare 6 lots tests : un lot contrôle (cellules non stimulées), un lot témoin KGF (cellules stimulées par EHL à une concentration de 3nM soit 90 ng/ml) et 4 lots traités (cellules stimulées par EHL et traitées avec le mélange de madécassoside et de terminoloside). On mesure les densités otiques à t=0 jours, t=2 jours et t=5 jours. Les moyennes des densités optiques, D.O., sont converties en densités cellulaires, cellules/puits, à partir de la droite de régression : D.O.=0,00458×($10^3$ cellules/puits) + 0,041

Le tableau 5 suivant regroupe les densités cellulaires enregistrées au bout de 2 et 5 jours après activation des cellules par l'EHL.

Tableau 5

| | Traitement 2 jours | | Traitement 5 jours | |
|---|---|---|---|---|
| Stimulation EHL | Cellules/puits | Croissance cellulaire | Cellules/puits | Croissance cellulaire |
| Témoin sans EHL | 66668 +/-1380 | | 89180 +/- 2202 | |
| Témoin avec EHL | 875973 +/- 1400 | 100% | 157616 +/-6359 | 100% |
| $C_1$ et EHL | 76764 +/- 799 | 101% n.s. | 114584 +/- 2463 | 100% n.s. |
| $C_2$ et EHL | 75891 +/- 1190 | 100% n.s. | 115539 +/- 1261 | 101% n.s. |
| $C_3$ et EHLF | 73408 +/-1690 | 97% (p ≤0,05) | 112428 +/- 1548 | 98% (p ≤0,05) |
| $C_4$ et EHL | 72016 +/- 1597 | 95% (p ≤0,05) | 93355 +/- 1429 | 82% p ≤0,05) |
| L'abréviation n.s. signifie que le résultat n'est pas statistiquement significatif.<br>p ≤0,05 signifie que les résultats sont statistiquement significatifs, avec une erreur de 0,05%. | | | | |

Les résultats obtenus montrent une légère diminution de la croissance des cellules HaCaT stimulées par l'EHL, après 2 ou 5 jours de culture en présence du mélange selon l'invention. Ce léger effet inhibiteur n'apparaît qu'aux fortes concentrations. A 300µg/ml, le mélange de madécassoside et de terminoloside réduit de 18% par rapport au témoin la croissance des cellules activées par l'EHL après 5 jours de traitement.

**c) Conclusion :**

En conclusion, le KGF, à une concentration de 100 ng/ml, et l'EHL, à une concentration de 90ng/ml, stimulent la croissance des cellules HaCaT.

Le mélange de madécassoside et de terminoloside n'est pas capable, dans la gamme des concentrations testées, de moduler la croissance des cellules HaCaT stimulées par le KGF. Le mélange selon l'invention est capable de moduler, de manière dose-dépendante, la croissance des cellules HaCaT stimulées par l'EHL. Le mélange de madécassoside et de terminoloside à la dose de 300µg/ml est capable de réduire de 18% la croissance des cellules HaCaT stimulées par l'EHL.

**Exemple 7: mise en évidence de l'activité anti-inflammatoire du mélange de madécassoside et de terminoloside sur des kératinocytes humains en culture :**

Le sujet de cet exemple est d'évaluer l'activité anti-inflammatoire du mélange selon l'invention sur la production et la libération de cytokines épidermique pro-inflammatoires ; IL-1α et PGE-2, par des kératinocytes humains en culture, soumis à un stress irritatif.

**a) Etude de la cytotoxicité du mélange de madécassoside et de terminoloside**

Cette étude de cytotoxicité a été menée de la même manière que dans l'exemple 4 et les mêmes résultats ont été obtenus. De plus, l'étude de cytotoxicité a été complétée par un essai sur kératinocytes humains, souche $K_{02-1}H_4$, afin de confirmer la non-toxicité des concentrations sur les cellules cibles.

Une gamme de 8 concentrations, de 0,075 à 5 mg/ml, a été étudiée. Le mélange de madécassoside et de terminoloside préalablement solubilise dans du DMSO a été ajouté au milieu de culture aux concentrations choisies, la concentration finale en DMSO est de 1%. La viabilité cellulaire a été effectuée par un test Rouge Neutre.

Les résultats confirment la non toxicité des concentrations inférieures ou égales à 2,5 mg/ml, après 72 heures

d'incubation. La plus forte concentration testée, de 5 mg/ml, entraîne une inhibition de 30% de la capture du Rouge Neutre.

Le tableau 6 ci-dessous regroupe les résultats. La viabilité cellulaire est exprimée en pourcentage de l'essai témoin.

Tableau 6

| Concentration (mg/ml) | 0,075 | 0,1 | 0,25 | 0,5 | 0,75 | 1 | 2,5 | 5 |
|---|---|---|---|---|---|---|---|---|
| Viabilité (72h) | 98% | 96% | 102% | 96% | 93% | 98% | 95% | 70% |

Compte tenu de ces résultats, on a décidé de retenir la concentration de 1mg/ml, comme dose maximale, pour tester l'activité anti-inflammatoire du mélange de madécassoside et de terminoloside.

**b) Activité anti-inflammatoire :**

Le principe du test repose sur l'évaluation de la production de cytokines pro-inflammatoires par des kératinocytes humains, en réponse à un stress irritatif. La méthode est basée sur la mesure du taux intracellulaire d'IL-1$\alpha$ et du taux de PGE-2 libéré dans le milieu extracellulaire par des kératinocytes cultivés en absence et en présence du mélange de madécassoside et de terminoloside et stimulé par le PMA, c'est-à-dire par le phorbol-1,2-myristate 13-acétate.

L'étude a été réalisée sur des kératinocytes isolés à partir de peau de prépuce de jeunes enfants, souche $K_{02\text{-}1}H_4$. 3 concentrations du mélange de madécassoside et de terminoloside ont été testées :

$C_1$=0,1 mg/ml $C_2$=0,5 mg/ml $C_3$=1,0 mg/ml

<u>b.1) Libération de PGE-2 :</u>

Le tableau 7 suivant regroupe les taux de PGE-2, exprimés en pg/$\mu$g de protéine, obtenu après activation des cellules ainsi que les taux basaux. La production de PGE-2 induite par le PMA et l'activité anti-inflammatoire (par la suite dénommée AAI) ont été calculés pour chaque concentration selon :

$$\text{PGE-2 production} = \text{PGE-2}_{\text{avec PMA}} - \text{PGE-2}_{\text{sans PMA}}$$

et

$$\text{AAI} = [(\text{PGE-2production}_{\text{cellules témoins}} - \text{PGE-2production}_{\text{cellules traitées}})/\text{PGE-2production}_{\text{cellules témoins}}]\times 100$$

Tableau 7

| mélange de madécassoside et de terminoloside | 0 mg/ml cultures témoins | 0,1 mg/ml | 0,5 mg/ml | 1 mg/ml |
|---|---|---|---|---|
| PGE-2 sans PMA | 4,96 +/- 0,52 | 4,96 +/- 0,52 | 4,96+/-0,52 | 4,96 +/- 0,52 |
| PGE-2 avec PMA | 39,34 +/-2,64 | 18,73 +/- 0,67 | 10,69 +/- 0,50 | 6,60 +/- 0,44 |
| PGE-2 production | 34,38 +/- 2,64 | 13,77 +/- 0,67 | 5,73 +/- 0,50 | 1,64 +/- 0,44 |
| Activité anti-inflammatoire (%) | | 60% | 83% | 95% |

L'exposition des kératinocytes au PMA se traduit par une très importante production et libération de PGE-2 dans les milieux de culture des cultures témoins. En effet, le taux de base enregistré au niveau des cultures non stimulées est multiplié par 8 après stimulation par le PMA. Ces résultats confirment que PGE-2 est une cytokine dont l'expression, la production et la libération peuvent être induites par un agent inflammatoire comme le PMA.

Les taux de PGE-2 enregistrés après stimulation par le PMA au niveau des cultures traitées avec le mélange de madécassoside et de terminoloside sont très nettement inférieurs à ceux observés au niveau des cultures témoins.

Cet effet inhibiteur du mélange selon l'invention vis-à-vis de la libération du PGE-2 induite par le PMA est dose-dépendant. Les différences enregistrées au niveau des lots $C_1$, $C_2$, et $C_3$ se sont révélées statistiquement significative ($p \leq 0{,}01$, test t de Student). La plus forte concentration du mélange de madécassoside et de terminoloside testée, de 1 mg/ml, est capable d'inhiber totalement la production de PGE-2 induite par le PMA.

b.2) Production d'IL-1α intracellulaire :

Le tableau 8 suivant regroupe les taux intracellulaires d'1L-1α, exprimés en pg/µg de protéine, obtenu après activation des cellules ainsi que les taux basaux. La production de d'IL-1α induite par le PMA et l'activité anti-inflammatoire (par la suite dénommée AAI) ont été calculés pour chaque concentration selon :

$$\text{IL-1}\alpha\,\text{production} = \text{IL-1}\alpha_{\text{avec PMA}} - \text{IL-1}\alpha_{\text{sans PMA}}$$

$$\text{et AAI} = [(\text{IL-1}\alpha\,\text{production}_{\text{cellules témoins}} - \text{IL-1}\alpha\,\text{production}_{\text{cellules traitées}}) / \text{IL-1}\alpha\,\text{production}_{\text{cellules témoins}}] \times 100$$

Tableau 8

| mélange de madécassoside, et de terminoloside | 0 mg/ml cultures témoins | 0,1 mg/ml | 0,5 mg/ml | 1 mg/ml |
|---|---|---|---|---|
| IL-1α sans PMA | 2,88 +/-.0,23 | 2,88+/-0,23 | 2,88 +/- 0,23 | 2,88 +/- 0,23 |
| IL-1α avec PMA | 12,11 +/- 0,41 | 8,94 +/- 0,11 | 6,86 +/-0,47 | 4,70 +/- 0,30 |
| IL-1α production | 9,23 +/- 0,41 | 6,06 +/- 0,11 | 3,98 +/- 0,47 | 1,82+/-0,30 |
| Activité anti-inflammatoire (%) | | 34% | 57% | 80% |

L'exposition des kératinocytes au PMA se traduit par une très importante production et libération d'IL-1α dans les milieux de culture des cultures témoins. En effet, le taux de base enregistré au niveau des cultures non stimulées est multiplié par 4 après stimulation par le PMA. Ces résultats confirment que IL-1α est une cytokine dont l'expression, la production et la libération peuvent être induites par un agent inflammatoire comme le PMA.

Les taux d'IL-1α enregistrés après stimulation par le PMA au niveau des cultures traitées avec le mélange de madécassoside et de terminoloside sont nettement inférieurs à ceux observés au niveau des cultures témoins. Cet effet inhibiteur du mélange selon l'invention vis-à-vis de la production et de l'accumulation d'IL-1α induite par le PMA est dose-dépendant. Les différences enregistrées au niveau des lots $C_1$, $C_2$, et $C_3$ se sont révélées statistiquement significative ($p \leq 0{,}01$, test t de Sondent). La plus forte concentration du mélange de madécassoside et de terminoloside testée, de 1 mg/ml, est capable de réduire de 80% la production d'IL-1α induite par le PMA.

**c) Conclusion :**

L'activité anti-inflammatoire du mélange de madécassoside et de terminoloside a été évaluée sur un modèle in vitro par sa capacité à moduler la production de deux cytokines épidermiques, PGE-2 et IL-1α, qui jouent un rôle clé dans les étapes du processus inflammatoire. L'étude a été réalisée sur des cultures de kératinocytes humains normaux stimulés par un agent irritant, le PMA. Cet exemple montre que le PMA, à une dose non toxique de 10ng/ml, induit, d'une part, une très nette augmentation du taux d'IL-1α intracellulaire et provoque, d'autre part, la production et la libération de PGE-2 dans les milieux de culture.

Le mélange de madécassoside et de terminoloside est capable de réduire très nettement la libération du PGE-2 induite par le PMA. Le mélange à la dose de-1 mg/ml est capable d'inhiber totalement la libération de PGE-2. Le mélange selon l'invention est capable de moduler, de manière dose-dépendante, la production et l'accumulation intracellulaire d'IL-1α induite par le PMA.

Compte tenu du rôle important de ces deux cytokines dans le processus inflammatoire, la capacité manifestée par le mélange de madécassoside et de terminoloside à moduler, de manière dose-dépendante, l'expression et la libération de PGE-2 ainsi que la production d'IL-1α par des kératinocytes stimulés in vitro, peut être considérée comme une activité anti-inflammatoire.

**Exemple 8 : évaluation de l'activité anti-inflammatoire du mélange de madécassoside et de terminoloside sur des kératinocytes humains en culture :**

Le sujet de cet exemple est d'évaluer l'activité anti-inflammatoire du mélange de madécassoside et de terminoloside sur la production de TNF-α et de IL-8 induite par l'interféron γ.

**a) Etude de cytotoxicité**

Cette étude de cytotoxicité a été menée de la même manière que dans l'exemple 4 et les mêmes résultats ont été obtenus.

Compte tenu de ces résultats, on a décidé de retenir la concentration de 1mg/ml, comme dose maximale, pour tester l'activité anti-inflammatoire du mélange de madécassoside et de terminoloside.

**b) Effet de l'interféron-γ sur la production de cytokines épidermiques**

L'étude est réalisée sur des kératinocytes humains normaux. Les cellules sont d'abord traitées avec 4 concentrations d'interféron-gamma (IFN-γ), pendant 24 heures.

Ensuite, on mesure les médiateurs de l'inflammation au niveau des cultures témoins et traitées par IFN-γ par un dosage d'IL-8 et de TNF-α dans les surnageants de culture et par un dosage des protéines cellulaires. La méthode de mesure utilisée est le test au bleu de Coomassie, c'est une méthode normalisée. Les résultats sont donnés dans les tableaux 9 et 10 suivants

Tableau 9

| IL-8 (pg/μg protéines) | Témoin | Interféron-γ (U/ml) | | | |
|---|---|---|---|---|---|
| | 0 | 100 | 500 | 1000 | 2000 |
| Moyenne<br>Ecart-type | 0,077<br>0,060 | 0,980<br>0,119 | 1,301<br>0,102 | 1,991<br>0,113 | 2,917<br>0,138 |

Tableau 10

| TNF-α (pg/μg protéines) | Témoin | Interféron-γ (U/ml) | | | |
|---|---|---|---|---|---|
| | 0 | 100 | 500 | 1000 | 2000 |
| Moyenne<br>Ecart-type | 3,260<br>0,519 | 3,198<br>0,744 | 8,605<br>1,195 | 10,773<br>1,673 | 14,545<br>1,256 |

Les résultats obtenus montrent que l'interféron-γ, dans la gamme des concentrations testées, de 100 à 2000 U/ml, induit, de manière dose-dépendante, une nette stimulation de la production de TNF-α et de IL-8. La lettre U est l'abréviation d'unité ; 1 U correspond à une unité d'interféron-γ.

Dans le cas de la cytokine épidermique TNF-α, le taux de base enregistré au niveau des cultures non stimulées est multiplié respectivement par 3.3 et par 4.5 après stimulation par l'interféron à 1000 et 2000 U/ml. Dans le cas de la cytokine épidermique IL-8, le taux de base enregistré au niveau des cultures non stimulées est multiplié respectivement par 26 et par 38 après stimulation par l'interféron à 1000 et 2000 U/ml.

Ces résultats confirment que l'interféron est capable de stimuler l'expression, la production et la libération de ces deux cytokines épidermique. Au vu de ces résultats, on a décidé de retenir la concentration 1000 U/ml pour stimuler les kératinocytes.

**c) Activité anti-inflammatoire du mélange de madécassoside et de terminoloside**

L'étude est réalisée sur des kératinocytes humains nominaux. Les cellules sont d'abord traitées, pendant 48 h, avec le produit à l'étude avant induction du stress irritatif. Ensuite, un stress irritatif est induit par addition d'une solution d'interféron-γ, IFN-γ. Les cellules ainsi activées sont incubées pendant 24h en présence du produit à l'étude. Enfin, on dose les taux d'IL-8 et de TNF-α dans les surnageants de culture et on dose aussi les protéines cellulaires.

c.1) Résultats sur la cytolone IL-8 :

Les tableaux suivants regroupent les taux d'IL-8, exprimé en pg/µg de protéines, obtenus après activation des cellules ainsi que le taux basal. L'activité anti-inflammatoire (AAI) a été calculée pour chaque concentration selon la formule :

$$A.A.I = [(IL\text{-}8_{(cellules\ témoins + IFN)} - IL\text{-}8_{(cellules\ traitées + IFN)}) / IL\text{-}8_{(cellules\ témoins + IFN)}]x100$$

Les résultats, des échantillons de kératinocytes traités avec le mélange de madécassoside et de terminoloside, sont donnés dans le tableau 11 suivant :

Tableau 11

| | Control sans IFN | Témoin avec IFN | IFN et mélange madécassoside + terminoloside | | | |
|---|---|---|---|---|---|---|
| | | 0 mg/ml | 0,1mg/ml | 0,25 mg/ml | 0,5 mg/ml | 1 mg/ml |
| IL-8 (pg/µg prot.) | 0,06 +/- 0,01 | 1,03 +/- 0,04 | 0,72+/- 0,09 | 0,53 +/- 0,06 | 0,39 +/- 0,03 | 0,39+/- 0,02 |
| Activité anti-inflammatoire | | | 31% | 49% | 62% | 63% |

Les résultats obtenus montrent une très importante production et libération de IL-8 dans les milieux des cultures témoins après stimulation par l'Interféron γ, à une concentration de 100U/ml. Le taux de base enregistré au niveau des cultures non stimulées est multiplié par 18 après stimulation par l'IFN-γ. D'autre part, ces résultats montrent aussi une très importante diminution, dose dépendante, du taux de IL-8 au niveau des cultures traitées stimulées par l'IFN-γ. La production de IL-8 induite par l'interféron est très nettement inférieure à celle observée au niveau des cultures du lot témoin. La plus forte concentration du mélange de madécassoside et de terminoloside est capable d'inhiber de 63% la libération de IL-8 induite par l'IFN-γ Les différences enregistrées au niveau des lots C1, C2, C3 et C4 se sont révélées statistiquement significatives (p≤0.01, test t de Student) par rapport au lot témoin.

c.2) Résultats sur la cytokine TNF-α

Les tableaux suivants regroupent les taux de TNF-α (pg/mg protéines) obtenus après activation des cellules ainsi que le taux basal L'activité anti-inflammatoire (AAI) a été calculée pour chaque concentration selon la formule :

$$A.A.I = [(TNF\text{-}\alpha_{(cellules\ témoins + IFN)} - TNF\text{-}\alpha_{(cellules\ traitées + IFN)}) / TNF\text{-}\alpha_{(cellules\ témoins + IFN)}]x100$$

Les résultats, des échantillons de kératinocytes traités avec le mélange de madécassoside et de terminoloside, sont donnés dans le tableau 12 suivant :

Tableau 12

| | Control sans IFN | Témoin avec IFN | IFN et mélange madécassoside + terminoloside | | | |
|---|---|---|---|---|---|---|
| | | 0 mg/ml | 0.0mg/ml | 0.25 mg/ml | 0.5 mg/ml | 1mg/ml |
| TNF-α (pg/µg prot.) | 2,39 +/- 0,16 | 5,26+/-0,31 | 4,95 +/- 0,22 | 4,97+/- 0,24 | 4,67+/- 0,15 | 3,92+/- 0,10 |
| Activité anti-inflammatoire | | | 6% | 6% | 11% | 26% |

Les résultats obtenus montrent une importante production et libération de TNF-α dans les milieux des cultures témoins après stimulation par l'Interféron γ, à une concentration de 100U/ml. Le taux de base enregistré au niveau des cultures non stimulées est multiplié par 2,2 après stimulation par l'IFN-γ, à une concentration de 1000 U/ml. Les résultats

obtenus montrent aussi une légère diminution, dose dépendante, du taux de TNF-α au niveau des cultures traitées stimulées par l'IFN-γ. La plus forte concentration du mélange de madécassosidc et de terminoloside est capable d'inhiber de 26% la libération de TNF-α induite par l'IFN-γ.

**d) Conclusion**

L'activité anti-inflammatoire du mélange de madécassoside et de terminoloside a été évaluée sur un modèle in vitro par sa capacité à moduler la production de deux cytokines épidermiques, IL-8 et TNF-α qui jouent un rôle clé dans les étapes du processus inflammatoire. L'étude a été réalisée sur des cultures de kératinocytes humains normaux stimulés par un agent irritant, l'IFN-γ.

Cet exemple montre que l'IFN-γ, à une dose non toxique de 1000 U/ml, induit, d'une part, une très nette augmentation du taux d'IL-8 et provoque, d'autre part, la production et la libération de TNF-γ dans les milieux de culture. Le mélange selon l'invention est capable de moduler, de manière dose-dépendante, la production et l'accumulation intracellulaire d'IL-8 induite par l'IFN-γ. Le mélange à la dose de 1 mg/ml est capable d'inhiber de 63% la libération de IL- 8.

Le mélange de madécassoside et de terminoloside est capable de réduire la libération du TNF-α induite par l'IFN-γ. Le mélange à la dose de 1 mg/ml est capable d'inhiber de 26% la libération de TNF-α.

Si nous devions extrapoler les résultats obtenus, en système clos, c'est-à-dire les tests effectués in-vitro, à des hypothèses de mécanisme d'activités cliniques effectives, in-vivo, nous pourrirons objectiver les observations suivantes : Le TNF-α est un des activateurs de la production d'IL8, non seulement au niveau kératinocytaire, mais aussi et surtout aux niveaux macrophagiques et lymphocytaires B.

Le fait que le mélange d'une part, ne diminue que partiellement l'expression du TNF-α, est, au delà de la modération de l'expression de signaux inflammatoires, la manifestation d'un contrôle de l'activité d'un système immunitaire non déprimé puisque TNF-α remplit un rôle majeur dans le contrôle et la surveillance cellulaires de la peau, notamment de la phase apoptotique. D'autre part ce mélange diminue l'expression d'IL8 avec pour conséquence une atténuation du chimiotactisme des polynucléaires neutrophiles et basophiles et les conséquences sur la diminution de la « pression » protéolytique, avec pour conséquence, la modération de la propagation du signal inflammatoire, et en outre, la diminution de la perméabilité vasculaire. Une autre conséquence est la moindre activation en retour des lymphocytes T de la peau par IL8.

**Exemple 9 : effet du mélange de madécassoside et de terminoloside vis-à-vis de la production de SKALP par des kératinocytes humains :**

Le sujet de cet exemple est d'évaluer l'activité anti-psoriasis du mélange de madécassoside et de terminoloside sur l'expression de la cytokératine 10, particulièrement de l'Elafin, ci après abrégées CK10 ou SKALP, par des kératinocytes en état de différenciation.

**a) Etude de cytotoxicité**

Cette étude de cytotoxicité a été menée de la même manière que dans l'exemple 4 et les mêmes résultats ont été obtenus. Compte tenu de ces résultats, on a décidé de retenir la concentration de 1mg/ml, comme dose maximale, pour tester l'activité anti-psoriasis du mélange de madécassoside et de terminoloside.

**b) Expression de SKALP et différenciation des kératinocytes**

L'étude est réalisée sur des kératinocytes humains normaux. Les cellules sont mises en culture dans trois milieux différents

- Milieu de « prolifération » : milieu KGM avec gf, growth factor ou facteur de croissance, milieu supplémenté en facteurs de croissance
- Milieu de «différenciation normale » : milieu KGM sans gf, milieu déplété en facteurs de croissance
- Milieu de « différenciation type psoriasis » : milieu KGM avec SVF, milieu supplémenté en sérum de veau foetal, ci après dénommé SVF (5%).

Ensuite, on mesure le taux de SKALP dans les milieux d'incubation des cellules cultivées pendant 72h dans ces différents milieux. Le test de mesure est le test ELISA décrit dans Skin pharmacollogy and applied skin physiology, 2002 ;15 :152-261.

Les résultats sont les suivants :

| | | |
|---|---|---|
| 1. Milieu KGM avec gf | SKALP = 1.17 +/- 0.1 ng/μg protéines | 100 |
| 2. Milieu KGM sans gf | SKALP = 1.34 +/- 0.20 ng/μg protéines | 115 |
| 3. Milieu KGM avec FCS | SKALP = 2.76 +/- 0.08 ng/μg protéines | 236 |

Les résultats obtenus montrent que l'expression de SKALP varie en fonction de l'état de différenciation des cellules. La production de SKALP est très nettement augmentée lorsque les cellules sont cultivées en milieu contenant du SVF. Le taux de SKALP en milieu de différenciation « type psoriasis » est multiplié par 2,4 par rapport au taux de SKALP enregistré en milieu de prolifération.

**c) Effet « anti-psoriasis » des produits étudiés**

Les conditions d'essai sont les suivantes: on met en culture des cellules en milieu de différenciation « normale », c'est-à-dire en milieu KGM sans gf, et en milieu de différenciation « type psoriasis »; c'est-à-dire en milieu KGM avec SVF, en absence, ce qui correspond au lot Témoin, et en présence du mélange selon l'invention, pendant 72h. On dose ensuite le taux de SKALP dans les surnageants de culture. On dose aussi les protéines cellulaires. Les tableaux 13 et 14 suivants regroupent les taux de SKALP, exprimés en ng/μg protéines, obtenus après culture des cellules en milieu KGM sans gf et en milieu KGM avec FCS.

*α) Culture en milieu de Différenciation « normale» : Milieu KGM sans gf*

Tableau 13

| | KGM avec gf | TCGM sans gf | KGM sans gf+ mélange selon l'invention | | | |
|---|---|---|---|---|---|---|
| | | 0 mg/ml | 0,1 mg/ml | 0,25 mg/ml | 0,5 mg/ml | 1mg/ml |
| SKALP (ng/μg prot.) | 1,17 +/- 0,18 | 1,34+/- 0,20 | 1,01 +/- 0,24 | 1,15 +/- 0,16 | 1,05 +/- 0,18 | 0,53 +/- 0,14 |
| SKALP PRODUCTION | | 100% | 75% | 86% | 78% | 40% |

Les résultats obtenus montrent une diminution du taux de SKALP au niveau des cultures traitées avec le mélange selon l'invention. Seule la différence enregistrée au niveau du lot C4 s'est révélée statistiquement significative ($p \leq 0.01$, test t de Student) par rapport au Témoin « KGM sans gf». La plus forte concentration du mélange de madécassoside et de terminoloside est capable d'inhiber de 60% la sécrétion de SKALP en milieu de différenciation « normale ».

β) *Culture en milieu de Différenciation « type Psoriasis»: Milieu KGM avec FCS*

Tableau 14

| | KGM avec gf | KGM avec FCS | KGM avec FCS + mélange selon l'invention | | | |
|---|---|---|---|---|---|---|
| | | 0 mg/ml | 0.1mg/ml | 0.25 mg/ml | 0.5 mg/ml | 1mg/ml |
| SKALP (ng/μg prot.) | 1,13 +/- 0,19 | 2,76+/- 0,08 | 2,22 +/- 0,24 | 2,34+/- 0,12 | 2,06 +/- 0,15 | 1,64+/- 0,10 |
| SKALP PRODUCTION | | 100% | 80% | 85% | 75% | 60% |

Les résultats obtenus montrent une diminution dose-dépendante du taux de SKALP au niveau des cultures traitées avec le mélange selon l'invention. Les différences enregistrées au niveau des lots C1, C2, C3 et C4 se sont révélées statistiquement significatives ($p \leq 0.01$, test t de Student) par rapport au Témoin « KGM avec FCS ». La plus forte concentration du mélange de madécassoside et de terminoloside est capable d'inhiber de 40% la sécrétion de SKALP en milieu de différenciation « type psoriasis ».

**d) Conclusion**

L'activité anti-psoriasis du mélange de madécassoside et de terminoloside a été évaluée sur un modèle in vitro par sa capacité à moduler la sécrétion de SKALP, qui jouerait un rôle clé dans les maladies du type psoriasis. L'étude a été réalisée sur des cultures de kératinocytes humains normaux dans différents milieux.

Cet exemple montre que l'expression de SKALP varie en fonction de l'état de différenciation des cellules et augmente très nettement dans un milieu «type psoriasis », c'est à dire contenant du FCS.

Le mélange selon l'invention est capable d'inhiber, de manière dose-dépendante, la sécrétion de SKALP en milieu de différenciation « normale » et en milieu de différenciation « type psoriasis ». Le mélange à la dose de 1 mg/ml est capable d'inhiber de 60% la sécrétion de SKALP en milieu de différenciation « normale » et de 40% la sécrétion de SKALP en milieu de différenciation« type psoriasis ». Compte tenu du rôle important de la sécrétion de SKALP dans le psoriasis, la capacité manifestée par le mélange de madécassoside et de terminoloside à moduler, de manière dose-dépendante, la sécrétion de SKALP par des kératinocytes dans différents milieux de différenciation in vitro, peut être considérée comme une activité anti-psoriasis. Ce résultat démontre l'effet protecteur du mélange selon l'invention vis à vis des cellules épidermiques dans le contexte protéolitique caractérisant un modèle de type psoriasis, selon un mode de désensibilisation cellulaire dans le contexte de la réaction hyperplasique inflammatoire.

**Exemple 10 : effets du mélange de madécassoside et de terminoloside sur l'activation de NFκB dans des cultures de fibroblastes :**

Le « Nuclear factor-κB » est un facteur de transcription qui gouverne l'expression des gènes codants les cytokines, les chimiokines, les facteurs de croissance, les molécules d'adhésion cellulaire et certaines protéines à phase brève. NFκB est activé par plusieurs agents, incluant les cytokines, les radicaux libres, les particules inhalées, l'irradiation ultraviolet, et des bactéries ou des virus.

Les agents inflammatoires tels que le TNF-α induisent la transcription de gènes pro-inflammatoires-cibles via l'activation, entre autres, du facteur de transcription NFκB. En conditions non stimulées, le facteur de transcription NFκB est lié dans le cytoplasme à une protéine inhibitrice Iκb. La liaison de TNF-α à son récepteur conduit à la phosphorylation de ce complexe et à la dissociation de NFκB de l'inhibiteur. NFκB activé migre alors dans le noyau et se fixe sur une séquence promotrice, appelée séquence consensus NFκB, spécifique des gènes trans-activés par NFκB. Cette séquence conduit à la transcription des gènes-cibles. L'activation de NFκB peut donc se mesurer en mettant en contact des extraits nucléaires stimulés ou non avec des séquences oligonucléotidiques consensus NFκB immobilisées sur des supports et en quantifiant NFκB lié à l'aide d'une méthode immuno-enzymatique, telle que ENLISA avec anticorps anti-NFκB.

Cette étude permet d'évaluer les effets du mélange selon l'invention sur l'activation de NFκB en utilisant une méthode spécifique très sensible basée sur la mesure de la liaison du facteur de transcription NFκB à la séquence oligonucléotidique consensus immobilisée sur support plastique. NFκB lié est secondairement reconnu par un anticorps spécifique anti-NFκB.

Cet essai a été réalisé sur des noyaux isolés de fibroblastes humains normaux.

Le milieu de culture est le DMEM qui comprend :

- L-glutamille 2mM
- pénicilline 50 UI/ml streptomycine 50 µg/ml
- sérum de veau foetal 10 % (v/v) pour la pré-culture puis passage en milieu sans sérum

Tableau 15

| Produits à l'essai | Solution-stock | Dilution | Concentration finale testée |
|---|---|---|---|
| Mélange selon l'invention | 10 % dans milieu de culture | Milieu de culture | 0.01% |

Tableau 16

| Références | Solution-stock | Dilution | Concentration finale testée |
|---|---|---|---|
| **Dexaméthasone** (Sigma D1756) | $10^{-2}$ M en DMSO | Milieu de culture | 0.1 µM |

(suite)

| Références | Solution-stock | Dilution | Concentration finale testée |
|---|---|---|---|
| **Sulfasalazine** (Sigma S0883, PM 398.4) | 1 M en DMSO | Milieu de culture | 5 mM |

**a) Etude de la cytotoxicité du mélange de madécassoside et de terminoloside**

Cette étude de cytotoxicité a été menée de la même manière que dans l'exemple 4 et les mêmes résultats ont été obtenus. Compte tenu de ces résultats, on a décidé de retenir la concentration de 0,01%, comme dose maximale, pour tester l'activité anti-psoriasis du mélange de madécassoside et de terminoloside.

**b) Traitements, extractions et dosages**

Les fibroblastes ont été pré-cultivés en flacons de 175 $cm^2$ en milieu DMEM à 10 % en volume de SVF jusqu'à confluence, puis le milieu de culture a été remplacé par du milieu DMEM sans sérum. Les cellules ont ensuite été cultivées en présence des produits à l'essai ou des références pendant 1 heure. L'agent pro-inflammatoire « transforming growth factor alpha » (TNF-$\alpha$, à 25 ng/ml final; Sigma T0157) a ensuite été ajouté au milieu de culture et les cellules ont été à nouveau incubées pendant 1 heure à 37°C et 5 % de $CO_2$. Après incubation, les cellules ont été récoltées sur glace et les noyaux cellulaires des différents échantillons ont été isolés à l'aide du kit Sigma NUC-101 suivant le protocole préconisé par le fournisseur. La quantité de protéines de chaque extrait de noyaux a été déterminée à l'aide du kit de dosage Biorad 500-0116. La quantité de NF$\kappa$B activé, lié à l'oligonucléotide spécifique, a été mesurée sur une même quantité d'extrait nucléaire de chaque échantillon, soit 50 $\mu$l, dilué à moitié, après révélation à l'aide d'un anticorps spécifique anti-NF$\kappa$B. Ce dosage a été réalisé à l'aide du kit de dosage Mercury transfactor NF$\kappa$B, BD Biosciences K2058-1, selon le protocole préconisé par le fournisseur.

**c) Dosage de NF$\kappa$B**

Les résultats sont donnés dans le tableau 17 suivant :

Tableau 17

| Traitement | NF$\kappa$B (UA) | moyenne | % témoin | p |
|---|---|---|---|---|
| Témoin non traité | 10,71<br>8,39<br>8,90<br>10,19 | 9,55 | 100 | - |
| Contrôle sans TNF-$\alpha$ | 0,00<br>0,62<br>0,62<br>-0,21 | 0,26 | 3 | p<001 |
| Sulfasalazine 5mM | 4,93<br>4,01<br>2,88<br>4,93 | 4,19 | 44 | p<001 |
| Dexaméthasone 0,1 $\mu$M | 10,94<br>7,3<br>9,90<br>9,67 | 9,46 | 99 | p>0,05 |
| Mélange selon l'invention 0,01% | 10,16<br>6,79<br>9,63<br>9,63 | 9,05 | 95 | p>0,05 |

UA représente les unités arbitraires de NFκB dans l'extrait, c'est la différence entre la valeur mesurée et le bruit de fond.

Le taux basal de NFκB dans les noyaux de fibroblastes est très faible. Le traitement par le TNF-α à 25ng/ml a fortement activé le facteur de transcription NFκB, d'un facteur 37 environ par rapport au contrôle sans TNF-α. Des contrôles utilisant un excès d'oligonucléotide ont permis de montrer que la quasi-totalité de la réponse observée était spécifique de la liaison de NFκB. La sulfasalazine à 5 mM, inhibiteur de référence de la translocation de NFκB, a nettement réduit l'activation du facteur de transcription NFκB induite par le TNF-α, 44 % du témoin avec TNF-α, $p<0{,}01$.

La dexaméthasone à 0.1 μM, n'a pas inhibé la translocation nucléaire de NFκB induite par le TNF-α. En faits, la dexaméthazone réprime la trans-activation des gènes induite par NFκB, mais n'inhibe pas l'activation de NFκB et sa translocation vers le noyau. Il y a ainsi un effet inhibiteur de la transcription mais pas d'effet sur la translocation de NFκB ; l'action de la dexaméthazone a lieu au niveau nucléaire, via le récepteur aux glucocorticoïdes, elle est donc postérieure à l'action de la sulfasalazine.

Le produit selon l'invention testé à 0.01 % n'a pas modifié de façon significative l'activation de NFκB induite par TNF-α, 95 % du témoin. Ce résultat n'exclut pas une activité anti-inflammatoire via un autre mécanisme

**d) Conclusion**

La non ingérence du madécassoside et de son isomère dans l'activation du NFκB a pour conséquence la transcription autorisée des ligands, tels que les cytokines et les chimiokines, inflammatoires qui rendent la cellule opérationnelle dans un cas d'agression antigénique pathogène.

Le madécassoside et son isomère n'ont aucune interaction avec ou contre l'activation du NFκB produit par les fibroblastes. La modération des ligands inflammatoires dans les dérèglements auto-immuns, IL1, IL8, TNF-α PGE2 au niveau membranaire, indique que le madécassoside et son isomère agirait plus au niveau transductionnel (meilleure tolérance en cas de dérive inflammatoire), et au niveau des systèmes d'expression et/ou de synthèse des protéines, peptides, ou autres ligands (moindre expression intra-cellulaire des IL).

**<u>Exemple 11 évaluation in vitro des effets modulateurs de l'asiaticoside et d'un mélange de madécassoside et de terminoloside (50/50 p/p) sur le production de métalloprotéinases de la matrice extracellulaire par des fibroblastes humains cultivés dans un modèle de dermite équivalent :</u>**

L'objet de cet exemple est d'évaluer les effets modulateurs de l'asiaticoside et d'un mélange de madécassoside et de terminoloside (50/50 p/p) sur la protection des métalloprotéines (MMPs) et de leurs inhibiteurs spécifiques (TIMPS) par des fibroblastes de peau humaine cultivés dans un derme équivalent (lattis de collagène). Une approche expérimentale *in vitro,* basée sur la mesure des taux de MMP-1 et de T1MP-1 libérés dans les milieux de culture par des fibroblastes humains cultivés en absence et en présence des produits, et stimulés par le TNF-α est proposée. L'étude a été réalisée sur des cultures de fibroblastes humains établies à partir de biopsies cutanées selon la méthode des explants. Les fibroblastes sont cultivés, selon les techniques en usage au laboratoire, en milieu DMEM (commercialisé sous la dénomination INVITROGEN™ par la société Life Technologies) supplémenté en sérum de veau foetal (SVF 10%) et en antibiotiques, à 37% en atmosphère humide air $CO_2$ (95%-5%).

**<u>a) cytotoxicité</u>**

Cette étude de cytotoxicité a été menée de la même manière que dans l'exemple 6.

• Asiaticoside :

Une solution-mère du produit a été préparée préalablement dans du DMSO puis diluée dans du milieu de culture afin d'obtenir les différentes solutions-tests (concentration finale de DMSO: 1%). Une gamme large de 8 concentrations [de 5 à 1000μg/ml] a été étudiée dans un premier temps afin d'encadrer la Dose maximale Non Cytotoxique (D.N.C). Après 24h de contact, aucun effet cytotoxique significatif est enregistré dans la gamme des concentrations testées. A partir de ces résultats, une gamme restreinte comprise entre 10 et 1000μg/ ml a été établie afin de préciser la Dose maximale Non Cytotoxique (DNC) après 48h de contact. Les résultats (tableau 18) confirment la non toxicité des concentrations inférieures à 1 mg/ml après 48h d'incubation. La viabilité cellulaire est réduite seulement de 12% lorsque les cellules sont incubées en présence de l'actif à 1mg/ml.

Tableau 18

| ASIATICOSIDE (pg/ml) | 10 | 25 | 50 | 75 | 100 | 250 | 500 | 750 | 1000 |
|---|---|---|---|---|---|---|---|---|---|
| Viabilité (24h) | 98% | 101% | 101% | n.t. | 101% | 101% | 97% | n.t. | 93% |
| Viabilité (48h) | 108% | 107% | 106% | 104% | 102% | 99% | 93% | 90% | 88% |

• Mélange madécassoside-terminoloside :

Une solution-mère du produit a été préparée préalablement dans du DMSO puis diluée dans du milieu de culture afin d'obtenir les différentes solutions-tests (concentration finale de DMSO : 1%). Une gamme large de 8 concentrations (entre 0,005 et 100mg/ ml) a été étudiée sur fibroblastes humains afin d'encadrer la Dose maximale Non Cytotoxique (D.N.C). Après 24h de contact, les concentrations égales à 1mg/ml n'induisent aucune modification significative de la capture du Rouge Neutre. Au-delà, un effet cytotoxique dose- dépendant est enregistré. A partir de ces résultats, une gamme restreinte comprise entre 0.1 et 10mg/ ml a été établie afin de préciser la Dose maximale Non Cytotoxique (DNC).

Les résultats (tableau 19) confirment la non toxicité des concentrations égales à 1mg/ml, après 48h d'incubation.

Tableau 19

| Mélange madécassoside et terminoloside (mg/ml) | 0,05 | 0,1 | 0,25 | 0,5 | 0,75 | 1 | 2,5 | 5 | 7,5 | 10 |
|---|---|---|---|---|---|---|---|---|---|---|
| Viabilité (24h) | 98% | 98% | n.t. | 96% | n.t. | 94% | n.t | 68% | n.t. | 60% |
| Viabilité (48h) | n.t. | 101% | 96% | 97% | 99% | 99% | 99% | 86% | 71% | 47% |

A partir des résultats de cytotoxicité, la concentration «1mg/ml» a été retenue comme Dose Maximale Non Cytotoxique (DNC), pour tester les actifs asiaticoside et mélange madécassoside-terminoloside vis-à-vis de la production des MMPs.

b) production de métalloprotéinsases

Des fibroblastes de derme humain cultivés en monocouche sont détachés de leur support par trypsinisation et mis en suspension dans du milieu complet. Après numérisation, la suspension cellulaire est diluée dans du milieu de culture et ajoutée, dans des proportions définies, à un mélange de milieu de culture concentré (1,76x DMEM), de sérum de veau foetal (SVF) et de collagène type I (extrait de l'acide acétique à partir de tendons de queues de rat). Le mélange est ensuite coulé, à froid, dans des plaques 24 puits à raison de 1ml/puits. Les dermes équivalents sont ensuite placés à 37°C et incubés pendant 72h, en atmosphère air-$CO_2$ (95%/5%). Les gels se contractent progressivement sous l'action des fibroblastes.

72 heures après ensemencement, le milieu de culture est éliminé et remplacé par du milieu neuf contenant les différents produits à l'étude (traitement « Préventif »). Les gels sont replacés à l'étuve à 37°C et incubés, pendant 24 heures, en atmosphère air-$CO_2$ (95%/5%).

Après incubation, les plaques sont vidées. Les cultures sont rincées puis exposées à une solution de TNF-$\alpha$ (10ng/ml) en absence (cultures témoins) ou en présence (culture traitées) des différentes concentrations de chaque produit à l'étude. Après addition de TNF-$\alpha$ les cultures sont replacées à l'étuve à 37°C pendant 24h. A la fin de l'incubation, les milieux sont prélevés, centrifugés et aliquotés pour le dosage de MMP-1 et de TIMP-1.

L'étude a été réalisée sur des fibroblastes humains cultivés dans un gel tridimensionnel de collagène (derme équivalent).

➢ Conditions opératoires :

1. Traitement des cellules pendant 24h, avant induction de MMP-1 parle TNF-$\alpha$
2. Induction de MMP-1 par addition de TNF-$\alpha$ (10ng/ml). Incubation 24h.
3. Mesure de la viabilité cellulaire et des taux de MMP-1 dans les surnageants des cultures témoins et traitées.

• Asiaticoside :

L'asiaticoside a été testé à 3 concentrations:

$C_1$ = 0,2 mg/ml $C_2$ = 0,5 mg/ml $C_3$ = 1,0 mg/ml

➢ Viabilité des cellules (test Rouge Neutre)

Le traitement des cellules par le INF-α à 10ng/ml n'entraîne aucune modification de la viabilité cellulaire. Aucune diminution de la capture du Rouge Neutre n'est observée après exposition au TNF-α De même, la viabilité des cultures traitées avec l'asiaticoside n'est pas modifiée après traitement par le TNF-α

➢ Production de MMP-1

Le tableau 20 suivant regroupe les taux de MMP-1 (ng/ml) obtenus dans les surnageants de culture après activation des cellules (+TNF) ainsi que les taux basaux (-TNF).

Tableau 20

| | Production de MMP-1 (ng MMP-1/ml) | | | |
|---|---|---|---|---|
| | (-) TNF-α | | (+) TNF-α | |
| Gels de collagène traités avec | Moyenne | Ecart-type | Moyenne | Ecart-type |
| Témoin | 2,24 | 0,54 | 12,11 | 1,03 |
| Asiaticoside (0,2mg/ml) | Non mesuré | | 9,96 | 0,47 |
| Asiaticoside (0,5mg/ml) | non mesuré | | 6,18 | 0,15 |
| Asiaticoside (1,0mg/ml) | Non mesuré | | 3,40 | 0,21 |

Au niveau des cellules témoins, les résultats obtenus montrent que le TNF-α induit une nette stimulation de la production de MMP-1. Le taux basal enregistré au niveau des cellules non stimulées est multiplié par 503 après stimulation par le TNF-α

Au niveau des cellules traitées, les taux de MMP-1 enregistrés après stimulation par le TNF-α au niveau des cultures traitées avec l'asiaticoside sont inférieurs à celui observé au niveau du contrôle (+)TNF-α.

L'effet de l'asiaticoside vis-à-vis de la production de MMP-1 induite par TNF-α est dose-dépendant. Les différences enregistrées au niveau des lots $C_1$ ($p \leq 0{,}05$), $C_2$ ($p \leq 0{,}01$) et $C_3$ ($p \leq 0{,}01$) se sont révélées statistiquement significatives (test t de Student) par rapport au contrôle (+)TNF-α :

- ■ $C_1$: 18% de diminution du taux de MMP-1
- ■ $C_2$: 49% de diminution du taux de MMP-1
- ■ $C_3$ : 72% de diminution du taux de MMP-1
- ■ Mélange madécassoside-terminoloside

Le mélange madécassoside-terminoloside a été testé à 3 concentrations:

$C_1$ = 0,2 mg/ml $C_2$ = 0,5 mg/ml $C_3$= 1,0 mg/ml

➢ Viabilité des cellules (test Rouge Neutre)

La viabilité des cultures traitées avec le mélange madécassoside-terminoloside n'est pas modifiée après traitement par le TNF-α

➢ Production de MMP-1

Les résultats montrent une diminution dose-dépendante des taux de MMP-1 dans les surnageants des cultures traitées avec mélange madécassoside-terminoloside et stimulées par TNF-α Seules les différences enregistrées au niveau des lots traités $C_2$ et $C_3$ sont statistiquement significatives ($p \leq 0.01$, test t de Student) :

■ $C_1$ : 3% de diminution du taux de MMP-1
■ $C_2$ : 39% de diminution du taux de MMP-1
■ $C_3$ : 61 % de diminution du taux de MMP-1

Le tableau 21 suivant regroupe les taux de MMP-1 (ng/m1) obtenus dans les surnageants de culture après activation des cellules (+ TNF) ainsi que les taux basaux (- TNF).

Tableau 21

| | Production de MMP-1 (ng MMP-1/ml) | | | |
|---|---|---|---|---|
| | (-) TNF-α | | (+) TNF-α | |
| Gels de collagène, traités avec | Moyenne | Ecart-type | Moyenne | Ecart-type |
| Témoin | 2,24 | 0,54 | 12,11 | 1,03 |
| Mélange madécassoside-terminoloside (0,2 mg/ml) | Non mesuré | | 11,78 | 0,83 |
| Mélange madécassoside-terminoloside (0,5mg/ml) | Non mesuré | | 7,37 | 0,76 |
| Mélange madécassoside-terminoloside (1,0mg/ml) | Non mesuré | | 4,76 | 0,52 |

c) production de TIMT-1

Le taux de TIMP-1, inhibiteur spécifique de MMP-1 a été également : mesuré dans les surnageants des cultures traitées avec l'asiaticoside ou avec le mélange madécassoside-terminoloside. Chaque actif a été testé à 3 concentrations:

CI = 0,2 mg/ml    C2 = 0,5 mg/ml et    C3 = 1,0 mg/ml

Le tableau 22 suivant regroupe les taux de TIMP-1 (ng/ml) obtenus dans les surnageants de culture après activation des cellules (+ TNF) ainsi que les taux basaux (- TNF).

Tableau 22

| | Production de TIMP-1 (ng YIMP-1/ml) | | | |
|---|---|---|---|---|
| | (-) TNF-α | | (+) TNF-α | |
| Gels de collagène traités avec | Moyenne | Ecart-type | Moyenne | Ecart-type |
| Témoin | 51,20 | 1,22 | 48,90 | 2,16 |
| Asiaticoside (0,2mg/ml) | Non mesuré | | 51,01 | 1,51 |
| Asiaticoside (0,5mg/ml) | Non mesuré | | 50,36 | 1,67 |
| Asiaticoside (1,0mg/ml) | Non mesuré | | 48,82 | 2,22 |
| Mélange madécassoside-terminoloside (0,2mg/ml) | Non mesuré | | 48,63 | 1,18 |
| Mélange madécassoside-terminoloside (0,5mg/ml) | Non mesuré | | 46,98 | 2,02 |
| Mélange madécassoside-terminoloside (1,0mg/ml) | Non mesuré | | 48,76 | 0,92 |

Les résultats obtenus montrent que:

■ la production de TIMP-1 par des fibroblastes cultivés dans un gel de collagène n'est pas modifiée par le TNF-α.
■ les actifs constitués de l'asiaticoside et du mélange madécassoside-terminoloside n'exercent, dans la gamme des concentrations testées, aucun effet vis-à-vis de la production de TIMP-1.

d) conclusion :

Les effets modulateurs des actifs constitués de l'asiaticoside et du mélange madécassoside-terminoloside vis-à-vis de la production de métalloprotéinases (MMPs) et de leurs inhibiteurs spécifiques (TIMPs) ont été appréciés, *in vitro,* sur un modèle de derme humain. L'étude a été réalisée sur des fibroblastes humains normaux cultivés dans une

matrice tri- dimensionnelle de collagène. Les effets des actifs ont été évalués par la mesure des taux de MMP-1 et de TIMP-1 dans les surnageants de culture après activation des cellules par le TNF-α.

Dans les conditions expérimentales retenues, les résultats de cette étude ont montré que:

■ le TNF-α à dose non cytotoxique (10 ng/ml) induit une importante augmentation de la production de MMP-1. Par contre, la production de TIMP-I n'est pas modifiée après traitement des cellules par le TMF-α.
■ les actifs constitués de l'asiaticoside et du mélange madécassoside-terminoloside sont capables de réduire, de manière dose-dépendante, la production de MMP-1 induite par le TNF-α A la plus forte concentration testée (1mg/ml), ces deux actifs sont capables de réduire respectivement de 726/0 et 61% le taux de MMP-1 induit par les TNF-α
■ l'asiaticoside et le mélange madécassoside-terminoloside sont, dans la gamme des concentrations testées, sans effet vis-à-vis de la production de TIMP-I.

L'étude, réalisée sur des fibroblastes humains, permet d'observer sous le stimuli inflammatoire du TNF-α, un accroissement important (530%) de libération de MMP1 (collagénase) dans le milieu de culture. Le mélange madécassoside-terminoloside, comme l'asiaticoside diminuent significativement, de façon dose- dépendante la concentration de MMP1 (Mélange madécassoside-terminoloside = -39% à 500 micro- grammes / kg et asiaticoside = -49%). -la libération des inhibiteurs de MMP1, les TIMP et en particulier TIMP1, est non modifiée. Ce résultat est à rapprocher des études effectuées sur les SKALP (exemple 9), puisque en présence du mélange madécassoside-terminoloside, une haute concentration de leuco-protéases, n'accroît pas la sécrétion des inhibiteurs, mais diminue fortement celle des ligands pro-inflammatoires par les kératinocytes.

Cela implique également, que le mélange madécassoside-terminoloside et l'asiaticoside sont actifs sur les deux compartiments dermiques que sont l'épiderme et le derme.

**Exemple 12 : activité anti-inflammatoire du terminoloside, du madécassoside et du mélange d'hétérosides (madécassoside, terminoloside et asiaticoside):**

Dans cet exemple, le mélange d'hétérosides est constitué en poids, par rapport au poids total du mélange, de 40% de terminoloside, 40% de madécassoside et de 20% d'asiaticoside.

Afin d'évaluer l'activité anti-inflammatoire du terminoloside, du madécassoside et du mélange d'hétérosides, nous avons étudié les effets modulateurs de ces trois actifs vis-à-vis de la production et libération de cytokines épidermiques pro- inflammatoires (IL-1α IL-8, PGE-2) par des kératinocytes humains en culture, soumis à un stress irritatif induit soit par l'interféron-gamma (IFN-γ) soit par le PMA. Des kératinocytes humaines ont été isolés à partir de peau humaine. Les cellules sont cultivées en milieu KGM « serum free » (Keratinocyte Growth Medium, commercialisé par la société Clonetics®), selon les techniques habituelles mises en place au laboratoire, à 37°C en atmosphère humide air-$CO_2$ (95%-5%). Les cellules sont ensemencées dans des flacons de 25 $cm^2$ et sont passées régulièrement avant d'atteindre la confluence. Le principe du test repose sur l'évaluation de la production de cytokines pro-inflammatoires par des kératinocytes humains, en réponse à un stress irritatif La méthode est basée sur la mesure du taux intracellulaire d'IL-I-α et des taux de IL-8 et de PGE-2 libérées dans le milieu extracellulaire par des kératinocytes cultivés en absence et en présence des produits à l'étude, et stimulés soit par l'interféron-gamma (IFN-γ) soit par le PMA (phorbol-12-myristate 13-acetate).

a) cytotoxicité

Cette étude de cytotoxicité a été menée de la même manière que dans l'exemple 6 sur des kératinocytes humains HaCaT et sur des kératinocytes humains normaux.

● Terminoloside :

o Cytotoxicité sur kératinocytes humains HaCaT

Le tableau 23 ci-dessous regroupe l'ensemble des résultats (viabilité cellulaire en % du contrôle).

Tableau 23

| Concentration (mg/ml) | 0,05 | 0,1 | 0,25 | 0,5 | 0,75 | 1 | 2,5 | 5 | 7,5 | 10 |
|---|---|---|---|---|---|---|---|---|---|---|
| Viabilité (24h) | 105% | 104% | 105% | 105% | n.t. | 104% | 100% | 81% | n.t. | 73% |

(suite)

| | | | | | | | | | | |
|---|---|---|---|---|---|---|---|---|---|---|
| Viabilité (72h) | n.t | 100% | 101% | 101% | 103% | 102% | 103% | 93% | 86% | 69% |
| n.t : non testé | | | | | | | | | | |

Après 24h de contact, les concentrations ≤ 2,5mg/ml n'induisent aucune modification significative de la réponse cellulaire vis-à-vis du RN. Au-delà de 2,5mg/ml, un léger effet cytotoxique dose-dépendant est enregistré.

o Cytotoxicité sur kératinocytes humains

L'étude de cytotoxicité a été complétée par un essai sur kératinocytes humains (souche $K_{02-1}H_4$) afin de confirmer la non toxicité des concentrations sur les cellules « cibles ». Le tableau 24 ci-dessous regroupe l'ensemble des résultats (viabilité cellulaire en % du contrôle).

Tableau 24

| Concentration (mg/ml) | 0,25 | 0,5 | 0,75 | 1,0 | 2,5 | 5 | 10 |
|---|---|---|---|---|---|---|---|
| Viabilité (72h) | 101% | 103% | 103% | 102% | 99% | 96% | 69% |

Les résultats confirment la non toxicité des concentrations inférieures ou égales à 2.5mg/ml après 72h d'incubation.

■ madécassoside :

o Cytotoxicité sur kératinocytes humains HaCaT

Le tableau 25 ci-dessous regroupe l'ensemble des résultats :

Tableau 25

| Concentration (mg/ml) | 0,05 | 0,1 | 0,25 | 0,5 | 0,75 | 1 | 2.5 | 5 | 7.5 | 10 |
|---|---|---|---|---|---|---|---|---|---|---|
| Viabilité (24h) | 105% | 102% | 103% | 99% | n.t. | 100% | 85% | 75% | n.t. | 63% |
| Viabilité (72h) | n.t. | 101% | 102% | 102% | 101% | 101% | 92% | 76% | 71% | 56% |

Après 24h de contact, les concentrations inférieures à 1,0mg/ml n'induisent aucune modification significative de la réponse cellulaire vis-à-vis du RN. Au-delà de 1,0 mg/ml, un léger effet cytotoxique dose-dépendant est enregistré.

o Cytotoxicité sur kératinocytes humains

L'étude de cytotoxicité a été complétée par un essai sur kératinocytes humains (souche $K_{02\cdot\ 1}H_4$) afin de confirmer la non toxicité des concentrations sur les cellules « cibles ».

Le tableau 26 ci-dessous regroupe l'ensemble des résultats (viabilité cellulaire en % du contrôle).

Tableau 26

| Concentration (mg/ml) | 0,25 | 0,5 | 0,75 | 1,0 | 2,5 | 5 | 10 |
|---|---|---|---|---|---|---|---|
| Viabilité (72h) | 102% | 101% | 102% | 101% | 97% | 88% | 72% |

Les résultats confirment la non toxicité des concentrations inférieures à 2,5 mg/ml. Au-delà, un léger effet cytotoxique dose-dépendant est enregistré.

• hétérosides :

o Cytotoxicité sur kératinocytes humains HaCaT

Le tableau 27 regroupe l'ensemble des résultats (viabilité cellulaire en % du contrôle).

Tableau 27

| Concentration (mg/ml) | 0,1 | 0,25 | 0,5 | 0,75 | 1 | 2,5 | 5 | 7,5 | 10 |
|---|---|---|---|---|---|---|---|---|---|
| Viabilité (24h) | 102% | 103% | 99% | 100% | 85% | 85% | 75% | n.t. | 63% |
| Viabilité (72h) | 100% | 101% | 97% | 95% | 97% | 90% | 80% | 74% | 38% |

Après 24h de contact, les concentrations inférieures à 1,0 mg/ml n'induisent aucune modification significative de la réponse cellulaire vis-à-vis du RN. Au-delà de 1,0 mg/ml, un léger effet cytotoxique dose-dépendant est enregistré.

o Cytotoxicité sur kératinocytes humains

L'étude de cytotoxicité a été complétée par un essai sur kératinocytes humains (souche $K_{02-1}H_4$) afin de confirmer la non toxicité des concentrations sur les cellules « cibles ». Le tableau 28 regroupe l'ensemble des résultats (viabilité cellulaire en % du contrôle).

Tableau 28

| Concentration (mg/ml) | 0,1 | 0,25 | 0,5 | 0,75 | 1,0 | 2,5 | 5 | 10 |
|---|---|---|---|---|---|---|---|---|
| Viabilité (72h) | 97% | 97% | 99% | 93% | 88% | 64% | 34% | 12% |

Les résultats confirment la non toxicité des concentrations inférieures à 0,75 mg/ml. Au-delà, un léger effet cytotoxique dose-dépendant est enregistré. A partir de ces résultats, la concentration égale à 1 mg/ml a été retenue comme Dose Maximale pour tester l'activité anti-inflammatoire des trois actifs.

b) effet de l'interféron-γ

Les effets de l'interféron-gamma (IFN-γ) sur la production et libération de cytokines épidermiques ont été étudiés sur des kératinocytes humains normaux (souche $K_{02-1}H_4$). 4 concentrations de IFN-g ont été testées:

$C_1$=100 U/ml   $C_2$ = 500 U/ml   $C_3$ = 1000 U/ml   $C_4$ = 2000 U/ml

➢ Production d'1L-1α intracellulaires

Les résultats sont donnés dans le tableau 29 suivant :

Tableau 29

| IL-1α (pg/μg protéines) | Témoin | Interféron-γ (U/mL) | | | |
|---|---|---|---|---|---|
| | 0 | 100 | 500 | 1000 | 2000 |
| Moyenne | 3,25 | 6,80 | 8,29 | 11,21 | 12,48 |
| Ecart-type | 0,05 | 0,12 | 0,12 | 0,73 | 0,80 |

Les résultats obtenus montrent que l'interféron-γ, dans la gamme des concentrations testées, induit, de manière dose dépendante, une nette stimulation de la production d'IL-1α intracellulaire. Le taux basal enregistré au niveau des cellules non stimulées (3,25 +/- 0,05 pg/μg prot,) est multiplié respectivement par 2,6 et 3,8 après stimulation par IFN-γ à 1000 et 2000 pg/ml.

➢ Libération de IL-8

Les effets de l'IFN-γ sur la production et libération de IL-8 ont été étudiés dans les mêmes conditions expérimentales. Les résultats sont donnés dans le tableau 30 suivant :

Tableau 30

| IL-8 (pg/µg protéines) | Témoin | Interféron-γ (U/mL) | | | |
|---|---|---|---|---|---|
| | 0 | 100 | 500 | 1000 | 2000 |
| Moyenne | 0,093 | 0,252 | 0,350 | 0,516 | 0,671 |
| Ecart-type | 0,013 | 0,042 | 0,009 | 0,004 | 0,048 |

Les résultats obtenus au niveau des cultures non stimulées par l'IFN-γ (-IFN-γ) confirment l'absence ou la présence de très faible taux d'IL-8 dans les milieux d'incubation des kératinocytes à l'état basal. Par contre, une nette stimulation de la production et libération d'IL-8 est enregistrée après stimulation par l'IFN-γ Cette augmentation dose-dépendante du taux d'IL-8 intracellulaire au niveau des cultures (+IFNγ) confirme le rôle de cette cytokine pro-inflammatoire sur la production et libération de IL-8.

> Libération de PGE-2

Les effets de l'IFN-γ sur la production et libération de PGE-2 ont été étudiés dans les mêmes conditions expérimentales que précédemment décrites (tableau 31).

Tableau 31

| PGE-2 extracellulaire (pg/µg protéines) | Témoin | Interféron-γ (U/mL) | | | |
|---|---|---|---|---|---|
| | 0 | 100 | 500 | 1000 | 2000 |
| Moyenne | 10,49 | 3,60 | 3,45 | 3,48 | 3,80 |
| Ecart-type | 3,16 | 0,61 | 0,32 | 0,74 | 0,13 |

Les résultats montrent que l'IFNγ n'est pas capable de stimuler, dans la gamme des concentrations testées, la production et libération de PGE-2.

c) Activité anti-inflammatoire :

L'étude a été réalisée sur des kératinocytes isolés à partir de peau de prépuce de jeunes enfants (souche $K_{02-1}H_6$).

Chaque actif, terminoloside, madécassoside et hétérosides, a été testé à 4 concentrations:

$C_1$= 0,10 mg/ml $C_2$=0,25mg/ml $C_3$ = 0;50 mg/ml $C_4$ = 1,00 mg/ml

Conditions d'essai:

1. Traitement des cellules, pendant 48h, avec le produit à l'étude avant induction du stress irritatif ; 2. Induction du stress irritatif IFN-γ (1000 U/ml); 3. Incubation des cellules « activées », pendant 24h, en présence du produit à l'étude ;4. Dosage: IL-8 (extracellulaire) et IL-1α (intracellulaire). Dosage des protéines cellulaires.

➢ Libération de IL-8

Les tableaux 32 à 34 suivants regroupent les taux d'IL-8 obtenus après activation des cellules (+IFN-γ) ainsi que les taux basaux (-IFN- γ). La production d'IL-8 induite par l'IFN-γ et l'activité anti-inflammatoire (AA1) ont été calculées, pour chaque concentration d'actif, selon

$$A.A.I = [(IL\text{-}8_{(\text{cellules témoins + IFN})} - IL\text{-}8_{(\text{cellules traitées-IFN})}) / IL\text{-}8_{(\text{cellules témoins + IFN})}]x100$$

■ Terminoloside

Tableau 32

| | Control (-IFN-γ) | Témoin (+IFN-γ) | IFN-γ + TERMINOLOSIDE | | | |
|---|---|---|---|---|---|---|
| | - | 0 mg/ml | 0,1 mg/ml | 0,25 mg/ml | 0,5 mg/ml | 1 mg/ml |
| IL-8 (pg/μg prot) | 0,099 +/- 0,005 | 0,843 +/- 0,026 | 0,862 +1- 0,076 | 0,762 +/- 0,052 | 0,708+/- 0,030 | 0,492+/- 0,014 |
| Activité anti-inflammatoire (%) | | | 0% | 9% | 15% | 42% |

Une très importante libération d'IL-8 est observée au niveau des cultures témoins après simulation par l'IFN-γ Le très faible taux de base enregistré au niveau des cultures non stimulées indique que TL-8 n'est pas présente à l'état basal mais que son expression et sa libération peuvent être induites par l'IFN-γ Le taux de base est multiplié par 8,5 après stimulation par l'IFN-γ. Les taux d'IL-8 enregistrés après stimulation par l'IFN-γ au niveau des cultures traitées avec l'actif terminoloside sont intérieurs à celui du contrôle (+)IFN-γ.

L'effet de l'actif terminoloside vis-à-vis de la libération d'IL-8 induite par IFN-γ est dose-dépendant. Les différences enregistrées au niveau des lots $C_2$ ($p \leq 0{,}05$), $C_3$ ($p \leq 0{,}01$) et $C_4$ ($p \leq 0{,}01$) se sont révélées statistiquement significatives (test t de Student) par rapport au contrôle (+)IFN-γ. La plus forte concentration testée (1mg/ml) est capable de réduire de 42% la production d'IL-8 induite par l'IFN-γ.

■ Madécassoside :

Tableau 33

| | Control (-IFN-γ) | Témoin (+IFN-γ) | IFN-γ + MADECASSOSIDE | | | |
|---|---|---|---|---|---|---|
| | - | 0 mg/ml | 0, 1 mg/ml | 0,25 mg/ml | 0,5 mg/ml | 1 mg/ml |
| IL-8 (pg/μg prot) | 0,099 +/- 0,005 | 0,843 +/- 0,026 | 0,656 +/- 0,043 | 0,643 +/- 0,047 | 0,614+/- 0,039 | 0,368+/- 0,019 |
| Activité anti-inflammatoire (%) | | | 21% | 24% | 27% | 56% |

Les résultats montrent une diminution dose-dépendante d'IL-8 au niveau des lots traités avec le madécassoside et stimulés par l'IFN-γ Les différences au niveau des lots $C_1$, $C_2$, $C_3$ et $C_4$ sont statistiquement significatives ($p \leq 0{,}01$, test de Student). La concentration 1mg/ml est capable de réduire de 56% la production d'IL-8.

■ Hétérosides :

Tableau 34

| | Control (-IFN-γ) | Témoin (+IFN-γ) | IFN-γ + HETEROSIDES | | | |
|---|---|---|---|---|---|---|
| | - | 0 mg/ml | 0,1 mg/ml | 0,25 mg/ml | 0,5 mg/ml | 1 mg/ml |
| IL-8 (pg/μg prot) | 0,099+/- 0,005 | 0, 843 +/- 0,026 | 0,83+/- 0,04 | 0,63+/- 0,1 | 0,47 +/- 0,06 | 0,36+/- 0,01 |
| Activité anti-inflammatoire (%) | | | 1% | 25% | 44% | 57% |

Les résultats montrent une diminution dose-dépendante d'IL-8 au niveau des lots traités avec le mélange d'hétérosides (madécassoside, terminoloside et asiaticoside) et stimulés par l'IFN-γ Les différences au niveau des lots

$C_1$, $C_2$, $C_3$ et $C_4$ sont statistiquement significatives (p ≤0,01, test t de Student). La concentration 1mg/ml est capable de réduire de 57% la production d'IL-8.

➢ Production d'IL-1α intracellulaire

Les tableaux 34 à 36 suivants regroupent les taux intracellulaires d'IL-1α après activation des cellules (+IFN-γ) et le taux basal (-IFN-γ). La production d'IL-1α induite par l'IFN-γ et l'activité anti- inflammatoire (AAI) ont été calculées pour chaque concentration selon:

$$IL\text{-}1\alpha\ production = IL\text{-}1\alpha_{(+IFN)} - IL\text{-}1\alpha_{(-IFN)}$$

$$A.A.I = [(IL\text{-}1\alpha\ production_{(cellules\ témoins)} - IL\text{-}1\alpha\ production_{(cellules\ traitées)})/IL\text{-}1\alpha\ production(cellules\ témoins)] \times 100$$

■ Terminoloside

Tableau 35

| | Control (-IFN-γ) | Témoin (+IFN-γ) | IFN-γ + TERMINOLOSIDE | | | |
|---|---|---|---|---|---|---|
| | - | 0 mg/ml | 0,1 mg/ml | 0,25 mg/ml | 0,5 mg/ml | 1 mg/ml |
| IL-1α(pg/μg prot) | 2, 71 +/- 0,09 | 10,04+/- 0,44 | 8,78+/- 0,77 | 8,08+/- 0,43 | 7,31 +/- 0,38 | 5,02+/- 0,13 |
| IL-1α production | | 7,33 | 6,07 | 5,37 | 4,60 | 2,31 |
| Activité anti-inflammatoire (%) | | | 17% | 27% | 37% | 68% |

Au niveau du Lot Témoin, le taux de base d'IL-1α est multiplié par 3,7 après traitement par l'IFN-γ, confirmant que 1L-1α est une cytokine dont la production intracellulaire peut être induite par l'IFN-γ. Une nette diminution du taux d'IL-1α est enregistrée au niveau des cultures traitées avec l'actif Terminoloside. L'effet inhibiteur est dose-dépendant. Les différences au niveau de $C_1$ (p ≤0,05), $C_2$ (p ≤0,01), $C_3$ (p ≤0,01) et $C_4$ (p ≤0,01) sont statistiquement significatives (*test t* de Student) par rapport au Témoin (+)IFN-γ

L'actif à 1 mg/ml est capable de réduire de 68% la production d'IL-1α induite par IFN-γ.

■ Madécassoside

Tableau 36

| | Control (-IFN-γ) | Témoin (+IFN-γ) | IFN-γ + MADECASSOSSIDE | | | |
|---|---|---|---|---|---|---|
| | - | 0 mg/ml | 0,1 mg/ml | 0,25 mg/ml | 0,5 mg/ml | 1 mg/ml |
| IL-1α (pg/μg prot) | 2,71+/- 0,09 | 10,04+/- 0,44 | 8,23+/- 0,57 | 8,23+/- 0,64 | 8,07+/- 0,69 | 6,05+/- 0,37 |
| IL-1α production | | 7,33 | 5,52 | 5,52 | 5,36 | 3,34 |
| Activité anti-inflammatoire (%) | | | 25% | 25% | 27% | 54% |

Les résultats montrent une diminution de 54% (p ≤0,01) du taux d'IL-1α niveau des cultures traitées avec l'actif madécassoside à 1mg/ml. Par contre, les concentrations $C_1$, $C_2$ et $C_3$ semblent équivalentes: une réduction de 25% (p ≤0,01) du taux d'IL-1α est observée.

■ Hétérosides

Tableau 37

| | Control (-IFN-γ) | Témoin (+IFN-γ) | IFN-γ + HETEROSIDES | | | |
|---|---|---|---|---|---|---|
| | - | 0 mg/ml | 0,1 mg/ml | 0,25 mg/ml | 0,5 mg/ml | 1 mg/ml |
| IL-1α (pg/µg prot) | 2,711+/- 0,09 | 10,04 +/- 0,44 | 8,78 +/- 0,13 | 9,03 +/- 0,83 | 7,52+/- 0,38 | 5,56 +/- 0,39 |
| IL-1α production | | 7,33 | 6,07 | 6,32 | 4,81 | 2,85 |
| Activité anti-inflammatoire (%) | | | 17% | 14% | 34% | 61% |

Les résultats montrent une diminution de 61% ($p \leq 0{,}01$) du taux d'IL-1α niveau des cultures traitées avec le mélange d'hétérosides à 1mg/ml.

d) Activité anti-inflammatoire-stress PMA :

Compte tenu de l'absence d'effet de l'IFN-γ sur la production et libération de PGE-2, l'effet anti- inflammatoire des actifs a été étudié sur des kératinocytes humains normaux (souche $K_{02-1}H_6$) stimulés par le PMA. Chaque actif a été testé à 4 concentrations:

$C_1$ = 0,10 mg/ml  $C_2$ = 0,25 mg/ml  $C_3$ = 0,50 mg/ml  $C_4$ =1,00 mg/ml

Conditions d'essai:

1. Traitement des cellules, pendant 48h, avec le produit à l'étude avant induction du stress irritatif
2. Induction du stress irritatif : PMA (10ng/ml).
3. Incubation des cellules « activées », pendant 24h, en présence du produit à l'étude.
4. Dosage de PGE-2 (extracellulaire) et dosage des protéines cellulaires.

➢ Libération de PGE-2

Les tableaux 37 à 39 suivants regroupent les taux de PGE-2 (pg/µg prot.) obtenus après activation des cellules (+PMA) ainsi que les taux basaux (-PMA). La production de PGE-2 induite par le PMA et l'activité anti-inflammatoire (AAI) ont été calculées, pour chaque concentration, selon: PGE-2 production = [(PGE-$2_{(+pMA)}$-PGE-$2_{(-PMA)}$

$$A.A.I = [(PGE\text{-}2\ production_{(cellules\ témoins)} - PGE\text{-}2\ production_{(cellules\ traitées)} / PGE\text{-}2production_{(cellules\ témoins)}]x100$$

■ Terminoloside

Tableau 38

| | Contrôle (-PMA) | Témoin (+PMA) | PMA + Actif "TERMINOLOSIDE" | | | |
|---|---|---|---|---|---|---|
| | - | 0 mg/ml | 0,1 mg/ml | 0,25 mg/ml. | 0,5 mg/ml | 1 mg/ml |
| PGE-2 (pg/ µg prot) | 165,8 +/- 19,8 | 660,0 +1- 51,6 | 660,0+/- 29,9 | 606,80 +/- 63,6 | 445,7 +/- 16,7 | 321,0 +/- 21,1 |
| PGE-2 production | | 494,2 | 494,2 | 441,0 | 279,9 | 115,2 |
| Activité anti-inflammatoire (%) | | | 0% | 10,8% | 43,4% | 68,6% |

Au niveau du Lot Témoin, l'exposition des kératinocytes au PMA se traduit par une très importante production et libération de PGE-2 dans les milieux des cultures témoins. Le taux de base enregistré au niveau des cultures non stimulées est multiplié par 4 après stimulation par le PMA, confirmant que PGE-2 est une cytokine dont l'expression, la production et la libération peuvent être induites par le PMA. Les résultats montrent une diminution dose-dépendante de PGE-2 niveau des lots traités avec terminoloside et stimulés par le PMA. Les différences au niveau des lots $C_3$ et $C_4$ sont statistiquement significatives (p ≤0,01, test t de Student). La concentration 1mg/ml est capable de réduire de 69% la libération de PGE-2.

■ Madécassoside

Tableau 39

| | Contrôle (-PMA) | Témoin (+PMA) | PMA+MADECASSOSIDE | | | |
|---|---|---|---|---|---|---|
| | - | *0 mg/ml* | *0,1 mg/ml* | *0,25 mg/ml* | *0,5 mg/ml* | *1 mg/ml* |
| PGE-2 (pg/ µg prot) | 165,8+/- 19,8 | 660,0 +/- 51,6 | 622,7+/- 18,9 | 618,1+/- 20,7 | 436,3 +/- 32,1 | 348,3+/-17,8 |
| PGE-2 production | | 494,2 | 456,9 | 452,3 | 270,5 | 182,5 |
| Activité anti-inflammatoire (%) | | | 7,5% | 8,5% | 45,3% | 63,1% |

Une nette diminution du taux de PGE-2 est enregistrée au niveau des cultures traitées avec l'actif madécassoside.L'effet inhibiteur est dose- dépendant. Seules les différences au niveau de $C_3$ et $C_4$ sont statistiquement significatives (p ≤0.01, test t de Student) par rapport au Témoin (+)PMA. L'actif madécassoside à une concentration de 1 mg/ml est capable de réduire de 63% la production de PGE-2 induite par le PMA.

■ Hétérosides

Tableau 40

| | Contrôle (-PMA) | Témoin (+PMA) | PMA + HETEROSIDES | | | |
|---|---|---|---|---|---|---|
| | - | 0 mg/ml | 0,1 mg/ml | 0,25 mg/ml | 0,5 mg/ml | 1 mg/ml |
| PGE-2 (pg/ µg prot) | 165,8 +/- 19,8 | 660,0+/-51,6 | 646,5 +/- 33,8 | 515,1+/- 48,4 | 326,8+/- 15,5 | 142,1 +/- 6,6 |
| PGE-2 production | | 494,2 | 480,7 | 349,3 | 161,0 | 0 |
| Activité anti-inflammatoire (%) | | | 2,7% | 29,3% | 67,4% | 100% |

L'effet inhibiteur est dose-dépendant. Le mélange d'hétéroside à une concentration de 1 mg/ml est capable

d'inhiber totalement la production de PGE-2 induite par le PMA.

e) Conclusion

L'activité anti-inflammatoire des actifs terminoloside, madécassoside et hétérosides a été évaluée sur un modèle *in vitro* par leur capacité à moduler la production de cytokines épidermiques (IL-8, IL-1α et PGE-2) qui jouent un rôle clé dans les étapes du processus inflammatoire. L'étude a été, réalisée sur des kératinocytes humains normaux stimulés par l'interféron-γ (IFN-γ) ou par le PMA.

L'activité anti-inflammatoire des actifs étudiés a été' appréciée par la mesure des taux de :

- IL-8 et IL-1α après activation par l'IFN-γ.
- PGE-2 après stimulation par le PMA.

Dans les conditions expérimentales retenues, cette étude a montré que:

> ➢ l'IFN-γ, dans la gamme des concentrations testées (100 à 2000U/ml), induit, de manière dose-dépendante une nette stimulation de la production d'IL-1α (intracellulaire) et provoque la production et libération d'IL-8 dans les milieux de culture. Par contre, les mêmes concentrations d'IFN-γ ne sont pas capables de stimuler la libération de PGE-2.
> ➢ le PMA, à dose non toxique (10 ng/ml), provoque la libération de PGE-2 dans les milieux de culture.
> ➢ les actifs terminoloside, madécassoside et hétérosides sont capables de moduler la production des interleukines IL-8 et IL-1α ainsi que celle de la prostaglandine-2 en réponse au stress inflammatoire. La comparaison des taux de cytokines a mis en évidence des effets anti-inflammatoires variables selon les produits étudiés, le type de cytokine considéré et les changements observés:

• Vis-à-vis d'IL-8

Les trois actifs testés sont capables de moduler, de manière dose-dépendante, la production et la libération extracellulaire de cette cytokine.

Si l'on considère le taux de cytokine enregistré après traitement des cellules avec la plus forte concentration d'actif, les 3 produits testés peuvent être classés, en fonction de leur potentiel anti-inflammatoire, de la manière suivante:

$$\text{Hétérosides} > \text{Madécassoside} > \text{Terminoloside}$$

• Vis-à-vis d'IL-1α

Les trois actifs testés sont capables de réduire également la production et l'accumulation intracellulaire d'IL-1α induite par l'IFN-γ Les 3 produits testés peuvent être classés, en fonction de l'effet anti-inflammatoire maximal, de la manière suivante:

$$\text{Terminoloside} \geq \text{Hétérosides} \geq \text{Madécassoside}$$

• Vis-à-vis de PGE-2

Les 2 actifs, madécassoside et terminoloside, sont capables de réduire très nettement la libération de PGE-2 induite par le PMA. Le mélange d'hétérosides est capable d'inhiber totalement la production de PGE-2 induite par le PMA. Ces résultats permettent de classer les produits de la manière suivante:

$$\text{Hétérosides} > \text{Madécassoside} \approx \text{Terminoloside}$$

Compte tenu du rôle important de ces cytokines dans le processus inflammatoire, la capacité manifestée par les actifs terminoloside, madécassoside et hétérosides à moduler, de manière importante, la production des interleukines IL-8 et IL-1α, ainsi que celle de la prostaglandine-2 par des kératinocytes stimulés *in vitro,* peut être considérée comme

un élément très favorable à l'activité « anti-inflammatoire» recherchée.

Art antérier

Sahu NP, Roy SK, Mahato SB. Spectroscopic determination of structures of triterpenoid trisaccharides from Centella asiatica. Phyto chemistry 1989 vol. 28. N° 11, pp 2852-2854.

Brinkhaus B, Lindner M, Schuppan D, Hahn G. Chemical pharmacological and clinical profile of the East-Asian medical plant Centella asiatica. Phytomedicine, Vol. 7, N° 5, pp. 427-448.

## Revendications

1. Procédé de préparation d'un extrait de Centella asiatica comprenant un mélange de madécassoside, de terminoloside et d'asiaticoside, **caractérisé en ce qu'**il comprend les étapes suivantes :

   a) extraction des parties aériennes de Centella asiatica au moyen d'un solvant alcoolique ;
   b) passage sur résine anionique de la solution alcoolique obtenue à l'étape a) ;
   c) délipidation sélective par extraction liquide/liquide de l'éluat obtenu à l'étape b) ;
   d) concentration de la phase hydro-alcoolique délipidée jusqu'en phase aqueuse avec filtrations successives ;
   e) passage successif sur résine cationique puis sur résine anionique de la phase aqueuse obtenue à l'étape d) ;
   f) stabilisation de la phase aqueuse obtenue à l'étape e) par addition d'alcool et obtention d'un mélange comprenant du madécassoside, du terminoloside et de l'asiaticoside.

2. Procédé de préparation d'un extrait de Centella asiatica comprenant un mélange de madecassoside et de terminoloside, **caractérisé en ce qu'**il comprend les étapes suivantes :

   a) extraction des parties aériennes de Centella asiatica au moyen d'un solvant alcoolique ;
   b) passage sur résine anionique de la solution alcoolique obtenue à l'étape a) ;
   c) délipidation sélective par extraction liquide/liquide de l'éluat obtenu à l'étape b) ;
   d) concentration de la phase hydro-alcoolique délipidée jusqu'en phase aqueuse avec filtrations successives ;
   e) passage successif sur résine cationique puis sur résine anionique de la phase aqueuse obtenue à l'étape d) ;
   f) stabilisation de la phase aqueuse obtenue à l'étape e) par addition d'alcool ;
   g) chromatographie sélective de la phase hydro-alcoolique pré-purifiée obtenue à l'étape f) ; et
   h) récupération du mélange de madécassoside et de terminoloside dans sa forme finale.

3. Procédé d'extraction selon la revendication 1 ou 2, **caractérisé en ce que** la résine anionique utilisée à l'étape b) est une résine anionique forte avec des groupements fonctionnels du type ammonium quaternaire.

4. Procédé d'extraction selon l'une quelconque des revendications précédentes, **caractérisé en ce que** la résine cationique utilisée à l'étape e) est une résine cationique forte avec des groupements fonctionnels du type sulfonates.

5. Procédé d'extraction selon l'une quelconque des revendications précédentes, **caractérisé en ce que** la résine anionique utilisée à l'étape e) est une résine anionique forte avec des groupements fonctionnels du type ammonium quaternaire.

6. Procédé d'extraction selon l'une quelconque des revendications 2 à 5, **caractérisé en ce que** le solvant utilisé lors de la chromatographie sélective de l'étape g) est un mélange d'eau et d'éthanol dans des proportions eau/éthanol variant de 50/50 à 90/10 en volume / volume.

7. Procédé d'extraction selon l'une quelconque des revendications 2 à 6, **caractérisé en ce que** la phase stationnaire utilisée lors de la chromatographie sélective est une phase stationnaire apolaire, notamment une phase stationnaire constituée de silices apolaires greffées, les greffons apolaires ayant 2 à 18 atomes de carbone.

8. Procédé d'extraction selon l'une quelconque des revendications 2 à 7, **caractérisé en ce que** le mélange de madécassoside et de terminoloside est obtenu avec une pureté supérieure à 95% en masse par rapport au poids

de l'extrait.

**9.** Procédé d'extraction selon l'une quelconque des revendications 2 à 7, **caractérisé en ce que** le mélange de madécassoside et de terminoloside obtenu a un rapport massique madécassoside : terminoloside compris entre 30% et 70%, avantageusement entre 40% et 60%.

**10.** Procédé d'extraction selon l'une quelconque des revendications 2 à 9, **caractérisé en ce qu'**il comprend en outre, en parallèle, une étape de standardisation du mélange obtenu à l'étape f) par ajout d'une quantité appropriée d'un extrait comprenant plus de 95% en masse d'un mélange de madécassosside ou de terminoloside, dans lequel le mélange a avantageusement un rapport massique en madécassoside : totum compris entre 30% et 70%, plus avantageusement entre 40% et 60%, de façon à ce que l'extrait final ainsi obtenu ait une pureté comprise entre 90 et 98% en poids, par rapport au poids total de l'extrait final.

**11.** Utilisation d'un extrait de Centella asiatica, obtenu par le procédé selon l'une quelconque des revendications 2 à 10, pour la fabrication d'un médicament ayant une activité anti-inflammatoire.

**12.** Utilisation selon la revendication 11, **caractérisée en ce que** le médicament est destiné au traitement des maladies auto-immunes, des maladies inflammatoires chroniques, des maladies inflammatoires atopiques ou des maladies intestinales.

**13.** Utilisation selon la revendication 11 ou 12, **caractérisée en ce que** le médicament est destiné au traitement du psoriasis, du vitiligo, du pityriasis, des sclérodermies, des dermatoses bulleuses, de l'eczéma, de la dermatite atopique, de l'allergie ou de l'arthrite rhumatoïde.

**14.** Utilisation selon l'une quelconque des revendications 11 à 13, **caractérisée en ce que** le médicament est destiné à la prévention et au traitement des dérives inflammatoires chroniques liées au vieillissement et ses conséquences.

**15.** Utilisation selon la revendication 14, **caractérisée en ce que** le médicament est destiné à la prévention et au traitement des maladies choisies parmi les sensibilisations anaphylactiques, les anomalies pigmentaires de la peau, l'hypervascularisation dermique, les fissurations inflammatoires.

**16.** Utilisation selon la revendication 15, **caractérisée en ce que** le médicament est destiné à réguler l'homéostasie tissulaire dermique.

## Claims

**1.** Method for preparing an extract of Centella asiatica comprising a mixture of madecassoside, of terminoloside and of asiaticoside, **characterized in that** it comprises the following steps:

a) extraction of the parts of Centella asiatica that are above ground by means of an alcoholic solvent;
b) passage of the alcoholic solution obtained in step a), over anionic resin;
c) selective defatting by liquid/liquid extraction of the eluate obtained in step b);
d) concentration of the defatted aqueous-alcoholic phase to an aqueous phase with successive filtrations;
e) successive passage of the aqueous phase obtained in step d), over cationic resin and then over anionic resin;
f) stabilization of the aqueous phase obtained in step e) by addition of alcohol and obtaining of a mixture comprising madecassoside, terminoloside and asiaticoside.

**2.** Method for preparing an extract of Centella asiatica comprising a mixture of madecassoside and of terminoloside, **characterized in that** it comprises the following steps:

a) extraction of the parts of Centella asiatica that are above ground by means of an alcoholic solvent;
b) passage of the alcoholic solution obtained in step a), over anionic resin;
c) selective defatting by liquid/liquid extraction of the eluate obtained in step b);
d) concentration of the defatted aqueous-alcoholic phase to an aqueous phase with successive filtrations;
e) successive passage of the aqueous phase obtained in step d), over cationic resin and then.over anionic resin;
f) stabilization of the aqueous phase obtained in step e) by addition of alcohol;
g) selective chromatography of the prepurified aqueous-alcoholic phase obtained in step f); and

h) recovery of the mixture of madecassoside and of terminoloside in its final form.

3. Extraction method according to Claim 1 or 2, **characterized in that** the anionic resin used in step b) is a strong anionic resin with functional groups of the quaternary ammonium type.

4. Extraction method according to any one of the preceding claims, **characterized in that** the cationic resin used in step e) is a strong cationic resin with functional groups of the sulfonate type.

5. Extraction method according to any one of the preceding claims, **characterized in that** the anionic resin used in step e) is a strong anionic resin with functional groups of the quaternary ammonium type.

6. Extraction method according to any one of Claims 2 to 5, **characterized in that** the solvent used during the selective chromatography of step g) is a mixture of water and ethanol in water/ethanol proportions ranging from 50/50 to 90/10 volume by volume.

7. Extraction method according to any one of Claims 2 to 6, **characterized in that** the stationary phase used during the selective chromatography is an apolar stationary phase, in particular a stationary phase consisting of grafted apolar silicas, the apolar grafts having 2 to 18 carbon atoms.

8. Extraction method according to any one of Claims 2 to 7, **characterized in that** the mixture of madecassoside and of terminoloside is obtained with a purity greater than 95 wt% relative to the weight of the extract.

9. Extraction method according to any one of Claims 2 to 7, **characterized in that** the mixture of madecassoside and of terminoloside obtained has a madecassoside:terminoloside ratio by mass of between 30% and 70%, advantageously between 40% and 60%.

10. Extraction method according to any one of Claims 2 to 9, **characterized in that** it also comprises, in parallel, a step consisting of standardization of the mixture obtained in step f), by the addition of an appropriate amount of an extract comprising more than 95 wt% of a mixture of madecassoside and of terminoloside, in which the mixture advantageously has a madecassoside:whole ratio by mass of between 30% and 70%, more advantageously between 40% and 60%, such that the final extract thus obtained has a purity of between 90 and 98 wt%, relative to the total weight of the final extract.

11. Use of an extract of Centella asiatica, obtained by means of the method according to any one of Claims 2 to 10, for the manufacture of a medicinal product having an anti-inflammatory activity.

12. Use according to Claim 11, **characterized in that** the medicinal product is intended for the treatment of autoimmune diseases, chronic inflammatory diseases, atopic inflammatory diseases or bowel diseases.

13. Use according to Claim 11 or 12, **characterized in that** the medicinal product is intended for the treatment of psoriasis, vitiligo, pityriasis, scleroderma, bullous dermatoses, eczema, atopic dermatitis, allergy or rheumatoid arthritis.

14. Use according to any one of Claims 11 to 13, **characterized in that** the medicinal product is intended for the prevention and treatment of drifting toward chronic inflammation associated with aging and its consequences.

15. Use according to Claim 14, **characterized in that** the medicinal product is intended for the prevention and treatment of diseases chosen from anaphylactic sensitizations, pigmentary anomalies of the skin, dermal hypervascularization and inflammatory fissuring.

16. Use according to Claim 15, **characterized in that** the medicinal product is intended for regulating dermal tissue homeostasis.

## Patentansprüche

1. Verfahren zur Zubereitung eines Centella asiatica-Extrakts, ein Gemisch aus Madecassoside, Terminoloside und Asiaticoside umfassend, **dadurch gekennzeichnet, dass** es die folgenden Schritte umfasst:

a) Extraktion der oberirdischen Teile von Centella asiatica mittels eines alkoholischen Lösungsmittels,
b) Passage der in Schritt a) gewonnenen alkoholischen Lösung über anionisches Harz,
c) selektive Delipidierung durch Flüssigkeit/Flüssigkeit-Extraktion des in Schritt b) gewonnenen Eluats,
d) Konzentration der delipidierten hydro-alkoholischen Phase bis zur wässrigen Phase mit sukzessiven Filtrationen,
e) sukzessive Passage über kationisches Harz, danach über anionisches Harz der in Schritt d) gewonnenen wässrigen Phase,
f) Stabilisierung der in Schritt e) gewonnenen wässrigen Phase durch Hinzufügen von Alkohol und Herstellung eines Gemischs, das Madecassoside, Terminoloside und Asiaticoside umfasst.

2. Verfahren zur Zubereitung eines Centella asiatica-Extrakts, ein Gemisch aus Madecassoside, Terminoloside und Asiaticoside umfassend, **dadurch gekennzeichnet, dass** es die folgenden Schritte umfasst:

a) Extraktion der oberirdischen Teile von Centella asiatica mittels eines alkoholischen Lösungsmittels,
b) Passage, der in Schritt a) gewonnenen alkoholischen Lösung über anionisches Harz,
c) selektive Delipidierung durch Flüssigkeit/Flüssigkeit-Extraktion des in Schritt b) gewonnenen Eluats,
d) Konzentration der delipidierten hydro-alkoholischen Phase bis zur wässrigen Phase mit sukzessiven Filtrationen,
e) sukzessive Passage über kationisches Harz, danach über anionisches Harz der in Schritt d) gewonnenen wässrigen Phase,
f) Stabilisierung der in Schritt e) gewonnenen wässrigen Phase durch Hinzufügen von Alkohol,
g) selektive Chromatographie der in Schritt f) gewonnenen vorgereinigten hydro-alkoholischen Phase, und
h) Rückgewinnung des Gemischs aus Madecassoside und Terminoloside in seiner finalen Form.

3. Extraktionsverfahren nach Anspruch 1 oder 2, **dadurch gekennzeichnet, dass** das in Schritt b) verwendete anionische Harz ein starkes anionisches Harz mit Funktionsgruppen vom Typ quaternäres Ammonium ist.

4. Extraktionsverfahren nach einem der vorangehenden Ansprüche, **dadurch gekennzeichnet, dass** das in Schritt e) verwendete kationische Harz ein starkes kationisches Harz mit Funktionsgruppen vom Typ Sulfonate ist.

5. Extraktionsverfahren nach einem der vorangehenden Ansprüche, **dadurch gekennzeichnet, dass** das in Schritt e) verwendete anionische Harz ein starkes anionische Harz mit Funktionsgruppen vom Typ quaternäres Ammonium ist.

6. Extraktionsverfahren nach einem der Ansprüche 2 bis 5, **dadurch gekennzeichnet, dass** das bei der selektiven Chromatographie von Schritt g) verwendete Lösungsmittel ein Gemisch aus Wasser und Ethanol in Wasser-Ethanol-Verhältnissen ist, die im Volumen/Volumen von 50/50 bis 90/10 schwanken.

7. Extraktionsverfahren nach einem der Ansprüche 2 bis 6, **dadurch gekennzeichnet, dass** die bei der selektiven Chromatographie verwendete stationäre Phase eine apolare stationäre Phase ist, vor allem eine stationäre Phase, die aus aufpolymerisierten apolaren Siliziumdioxiden besteht, wobei die apolaren Pfropfe 2 bis 18 Kohlenstoffatome haben.

8. Extraktionsverfahren nach einem der Ansprüche 2 bis 7, **dadurch gekennzeichnet, dass** das Gemisch aus Madecassoside und Terminoloside mit einer Reinheit von über 95 % Massenanteil im Verhältnis zum Gewicht des Extrakts gewonnen wird.

9. Extraktionsverfahren nach einem der Ansprüche 2 bis 7, **dadurch gekennzeichnet, dass** das gewonnene Gemisch aus Madecassoside und Terminoloside ein Massenverhältnis Madecassoside : Terminoloside zwischen 30 % und 70 % inklusive hat, in vorteilhafter Weise zwischen 40 % und 60 %.

10. Extraktionsverfahren nach einem der Ansprüche 2 bis 9, **dadurch gekennzeichnet, dass** es weiterhin parallel einen Schritt der Standardisierung des in Schritt f) gewonnenen Gemischs umfasst durch Hinzufügen einer geeigneten Menge eines Extrakts, das mehr als 95 % Massenanteil eines Gemischs aus Madecassoside oder Terminoloside umfasst, wobei das Gemisch in vorteilhafter Weise ein Massenverhältnis Madecassoside : Totum zwischen 30 % und 70 % inklusive hat, in noch vorteilhafter Weise zwischen 40 % und 60 %, so dass der derart gewonnene finale Extrakt eine Reinheit zwischen 90 und 98 % Massenanteil inklusive im Verhältnis zum Gesamtgewicht des finalen Extrakts hat.

**11.** Verwendung eines Centella asiatica-Extrakts, der durch das Verfahren nach einem der Ansprüche 2 bis 10 gewonnen wurde, zur Herstellung eines Arzneimittels mit einer entzündungshemmenden Wirkung.

**12.** Verwendung nach Anspruch 11, **dadurch gekennzeichnet, dass** das Arzneimittel zur Behandlung von Autoimmunerkrankungen, chronischen entzündlichen Erkrankungen, atopischen entzündlichen Erkrankungen oder Darmerkrankungen bestimmt ist.

**13.** Verwendung nach Anspruch 11 oder 12, **dadurch gekennzeichnet, dass** das Arzneimittel zur Behandlung von Psoriasis, Vitiligo, Pityriasis, Sklerodermien, Bullosis, Ekzemen, atopischer Dermatitis, Allergie oder rheumatischer Arthritis bestimmt ist.

**14.** Verwendung nach einem der Ansprüche 11 bis 13, **dadurch gekennzeichnet, dass** das Arzneimittel zur Vorbeugung und zur Behandlung von chronischen entzündlichen Tendenzen bestimmt ist, die mit der Alterung und ihren Folgen verbunden sind.

**15.** Verwendung nach Anspruch 14, **dadurch gekennzeichnet, dass** das Arzneimittel zur Vorbeugung und zur Behandlung von Erkrankungen bestimmt ist, die aus den anaphylaktischen Sensibilisierungen, den Pigmentanomalien der Haut, der dermalen Hypervaskularisierung, den entzündlichen Fissuren ausgewählt sind.

**16.** Verwendung nach Anspruch 15, **dadurch gekennzeichnet, dass** das Arzneimittel zur Regulierung der dermalen Gewebshomöostase bestimmt ist.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- *Skin pharmacollogy and applied skin physiology,* 2002, vol. 15, 152-261
- **Sahu NP ; Roy SK ; Mahato SB.** Spectroscopic determination of structures of triterpenoid trisaccharides from Centella asiatica. *Phyto chemistry,* 1989, vol. 28 (11), 2852-2854
- **Brinkhaus B ; Lindner M ; Schuppan D ; Hahn G.** Chemical pharmacological and clinical profile of the East-Asian medical plant Centella asiatica. *Phytomedicine,* vol. 7 (5), 427-448